(19) Europäisches Patentamt
European Patent Office
Office européen des brevets

(11) EP 4 520 748 A1

(12) EUROPEAN PATENT APPLICATION
published in accordance with Art. 153(4) EPC

(43) Date of publication:
**12.03.2025 Bulletin 2025/11**

(21) Application number: **23803626.3**

(22) Date of filing: **12.05.2023**

(51) International Patent Classification (IPC):
***C07C 269/08*** (2006.01) ***C07C 227/28*** (2006.01)
***C07C 229/12*** (2006.01) ***C07C 271/22*** (2006.01)
***C07K 1/00*** (2006.01) ***C12N 9/06*** (2006.01)
***C12N 15/31*** (2006.01) ***C12N 15/53*** (2006.01)
***C12P 13/04*** (2006.01)

(52) Cooperative Patent Classification (CPC):
**C07C 227/28; C07C 229/12; C07C 269/08; C07C 271/22; C07K 1/00; C07K 14/195; C12N 9/0004; C12P 13/04;** Y02P 20/55

(86) International application number:
**PCT/JP2023/017844**

(87) International publication number:
**WO 2023/219152 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**13.05.2022 JP 2022079708**
**13.05.2022 JP 2022079712**
**23.12.2022 PCT/JP2022/047741**
**23.12.2022 EP 22216504**
**23.12.2022 TW 111149813**

(71) Applicant: **CHUGAI SEIYAKU KABUSHIKI KAISHA**
**Tokyo 115-8543 (JP)**

(72) Inventors:
- **EJIMA, Hirotaka**
  **Kamakura-shi, Kanagawa 247-8530 (JP)**
- **SHINODA, Kiyomichi**
  **Yokohama City, Kanagawa 244-8602 (JP)**

(74) Representative: **Vossius & Partner**
**Patentanwälte Rechtsanwälte mbB**
**Siebertstrasse 3**
**81675 München (DE)**

Remarks:
The complete document including Reference Table(s) and the Sequence Listing(s) can be downloaded from the EPO website

# (54) METHOD FOR PRODUCING SALT OF AMINO ACID OR SALT OF PEPTIDE COMPOUND OR SOLVATE OF EITHER ONE OF SAID SALTS COMPRISING LITHIUM SALT PRECIPITATION STEP

(57) One aspect of the present invention relates to a method for producing a salt of an amino acid or a salt of a peptide compound or a solvate thereof, comprising the following steps (A) and (B): step (A); a step of contacting a lithium-containing substance with a mixture to be purified which is a mixture of the following purification target (i) and the following impurities (ii): (i) the amino acid having a protective group at the N terminus or the peptide compound having a protective group at the N terminus, and (ii) compounds other than the purification target, and step (B); a step of precipitating a lithium salt of the purification target.

EP 4 520 748 A1

## Description

[Technical Field]

The present invention relates to a method for producing a salt of an amino acid or a salt of a peptide compound or a solvate thereof, comprising a step of precipitating a lithium salt.

[Background Art]

Salts of amino acids or salts of peptide compounds or solvates thereof are important compounds that can be candidate molecules for medicaments or synthetic intermediates thereof. As methods of purifying salts of amino acids or salts of peptide compounds or solvates thereof, methods are known in which they are protected or modified at the terminus, and then purified.

For example, Patent Literature 1 discloses a method in which an optically active 3-chloroalanine derivative is used, and an optically active aziridine-2-carboxylic acid derivative whose amino group is protected with a benzene sulfonyl group substituted with a nitro group at positions 2 and/or 4, or an optically active 3-haloalanine derivative whose amino group is protected with a benzene sulfonyl group substituted with a nitro group at positions 2 and/or 4 are formed as intermediates.

Also, Patent Literature 2 discloses a method for producing β-halogeno-α-aminocarboxylic acid or a salt thereof, characterized by halogenating a hydroxyl group by treating β-hydroxy-α-aminocarboxylic acid (provided that the basicity of the amino group at position α is not shielded by the presence of a substituent of the amino group) or a salt thereof with an acid, with a halogenating agent.

For a Fmoc group (9-fluorenylmethyloxycarbonyl group), which is widely used as a protective group, Non Patent Literature 1 shows that when a peptide compound is protected with the Fmoc group, Fmoc-β-alanine is produced as a by-product, and that Fmoc-β-alanine is derived from Fmoc-OSu (Fmoc N-hydroxysuccinimide ester). Fmoc-OSu is one of the starting materials commonly used in protecting amino acid or peptide compounds with an Fmoc group. Even when a generic protective group other than the Fmoc group is used (for example, a tert-butoxycarbonyl group (Boc group), a benzyloxycarbonyl group (Cbz group), or the like), β-alanine having the protective group may be generated as an impurity. It has thus been desired to improve the purity of candidate molecules for drug discovery or synthetic intermediates thereof by removing these impurities with high precision.

[Citation List]

[Patent Literature]

[Patent Literature 1] International Publication No. WO 2001/060795
[Patent Literature 2] International Publication No. WO 1999/033785

[Non Patent Literature]

[Non Patent Literature 1] M. Obkircher et al., "Formationof Fmoc-β-alanine during Fmoc-protections with Fmoc-Osu", Journal ofPeptide Science, Volume14, Issue6, June 2008, Pages 763-766

[Summary of Invention]

[Problems to be Solved by the Invention]

However, it has been difficult to reduce the content of impurities such as β-alanine or derivatives thereof, and purify the salt of the amino acid or the salt of the peptide compound or the solvate thereof of interest with high purity. Accordingly, an object of the present invention is to provide a salt of an amino acid or a salt of a peptide compound or a solvate thereof with high purity.

[Means for Solving the Problems]

[1] A method for producing a salt of an amino acid or a salt of a peptide compound or a solvate thereof, comprising the following steps (A) and (B):
step (A); a step of contacting a lithium-containing substance with a mixture to be purified which is a mixture of the following purification target (i) and the following impurities (ii):

(i) the amino acid having a protective group at the N terminus or the peptide compound having a protective group at the N terminus, and
(ii) compounds other than the purification target, and step (B); a step of precipitating a lithium salt of the purification target.

[2] A method for removing impurities from a mixture to be purified, comprising the following steps (A) and (B):
step (A); a step of contacting a lithium-containing substance with a mixture to be purified which is a mixture of the following purification target (i) and the following impurities (ii):

(i) the amino acid having a protective group at the N terminus or the peptide compound having a protective group at the N terminus, and
(ii) compounds other than the purification target, and step (B); a step of precipitating a lithium salt of the purification target.

[3] The method according to [1] or [2], wherein the purification target is a salt of an amino acid or a salt of a peptide compound or a solvate thereof.
[4] The method according to any one of [1] to [3], wherein the protective group at the N terminus of the purification target is a carbamate-type protective group.
[5] The method according to any one of [1] to [4], wherein the purification target has a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group.
[6] The method according to any one of [1] to [5], wherein the impurities are amino acids having a protective group at the N terminus or peptide compounds having a protective group at the N terminus, other than the purification target.
[7] The method according to any one of [1] to [6], wherein the impurities are β-alanine having a protective group at the N terminus.
[8] The method according to any one of [1] to [7], wherein the impurities have a carbamate group as a protective group.
[9] The method according to any one of [1] to [8], wherein the impurities have a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group as a protective group.
[10] The method according to any one of [1] to [9], wherein the purification target and the impurities have the same protective group.
[11] The method according to any one of [1] to [10], wherein the purification target and the impurities have the same protective group at the N terminus.
[12] The method according to any one of [1] to [11], wherein the lithium salt of the purification target is formed in the step (A).
[13] The method according to any one of [1] to [12], wherein a lithium salt of the impurities is formed in the step (A).
[14] The method according to any one of [1] to [13], wherein the lithium salt of the purification target or a solvate thereof is precipitated as a solid in the step (B).
[15] The method according to any one of [1] to [14], wherein the lithium salt of the purification target or a solvate thereof is precipitated as crystals in the step (B).
[16] The method according to any one of [1] to [15], wherein the step (A) is performed in the presence of a first organic solvent.
[17] The method according to [16], wherein the solubility of the purification target in the first organic solvent is 20 g/L or more.
[18] The method according to [16] or [17], wherein the solubility of the lithium salt of the purification target in the first organic solvent is 20 g/L or less.
[19] The method according to any one of [16] to [18], wherein the solubility of the impurities in the first solvent is 20 g/L or more.
[20] The method according to any one of [16] to [19], wherein the first organic solvent is at least one selected from the group consisting of nitriles, ethers, ketones, amides, ureas, sulfoxides, sulfones, alkanes, aromatic compounds, esters, and alkyl halides.
[21] The method according to any one of [16] to [20], wherein the first organic solvent is nitriles or ethers.
[22] The method according to any one of [16] to [21], wherein the first organic solvent is at least one selected from the

group consisting of acetonitrile, methanol, ethanol, 1-propanol, 2-propanol, 1-butanol, 2-butanol, 2-methyl-2-propanol, ethylene glycol, tetrahydrofuran, 1,4-dioxane, 2-methyltetrahydrofuran, methyl tert-butyl ether, diisopropyl ether, diethyl ether, 1,2-dimethoxyethane, acetone, methyl ethyl ketone, methyl isobutyl ketone, dimethylformamide, dimethylacetamide, N-methyl-2-pyrrolidone, 1,3-dimethyl-2-imidazolidinone, dimethyl sulfoxide, sulfolane, heptane, methylcyclohexane, hexane, cyclohexane, pentane, benzene, toluene, 1,2-dimethylbenzene, 1,3-dimethylbenzene, 1,4-dimethylbenzene, ethyl acetate, isopropyl acetate, dichloroethane, dichloromethane, chloroform, and carbon tetrachloride.

[23] The method according to any one of [16] to [22], wherein the first organic solvent is acetonitrile or tetrahydrofuran.

[24] The method according to any one of [16] to [23], wherein the first organic solvent is of only one type.

[25] The method according to any one of [1] to [24], wherein the step (A) is performed in the presence of water.

[26] The method according to any one of [16] to [25], wherein no organic solvent other than the first organic solvent is used in the step (A).

[27] The method according to any one of [1] to [26], wherein the step (B) is performed in the presence of a second organic solvent.

[28] The method according to [27], wherein the solubility of the lithium salt of the purification target in the second organic solvent is 10 g/L or less.

[29] The method according to [27] or [28], wherein the second organic solvent is at least one selected from the group consisting of nitriles, alcohols, ethers, ketones, amides, ureas, sulfoxides, sulfones, alkanes, aromatic compounds, esters, and alkyl halides.

[30] The method according to any one of [27] to [29], wherein the second organic solvent is nitriles or ethers.

[31] The method according to any one of [27] to [30], wherein the second organic solvent is at least one selected from the group consisting of acetonitrile, methanol, ethanol, 1-propanol, 2-propanol, 1-butanol, 2-butanol, 2-methyl-2-propanol, ethylene glycol, tetrahydrofuran, 1,4-dioxane, 2-methyltetrahydrofuran, methyl tert-butyl ether, diisopropyl ether, diethyl ether, 1,2-dimethoxyethane, acetone, methyl ethyl ketone, methyl isobutyl ketone, dimethylformamide, dimethylacetamide, N-methyl-2-pyrrolidone, 1,3-dimethyl-2-imidazolidinone, dimethyl sulfoxide, sulfolane, heptane, methylcyclohexane, hexane, cyclohexane, pentane, benzene, toluene, 1,2-dimethylbenzene, 1,3-dimethylbenzene, 1,4-dimethylbenzene, ethyl acetate, isopropyl acetate, dichloroethane, dichloromethane, chloroform, and carbon tetrachloride.

[32] The method according to any one of [27] to [31], wherein the second organic solvent is acetonitrile or tetrahydrofuran.

[33] The method according to any one of [27] to [32], wherein the second organic solvent is only one type.

[34] The method according to any one of [1] to [33], wherein the step (B) is performed in the presence of water.

[35] The method according to any one of [13] to [34], wherein the solubility of a lithium salt of the impurities in water is 10 g/L or more.

[36] The method according to any one of [27] to [35], wherein no organic solvent other than the second organic solvent is used in the step (B).

[37] The method according to any one of [27] to [36], wherein the sample obtained in the step (A) is mixed with the second solvent in the step (B).

[38] The method according to any one of [27] to [37], wherein the second organic solvent is further added in the step (B).

[39] The method according to [37] or [38], wherein the lithium salt of the purification target is precipitated by the step according to [37] or [38] above.

[40] The method according to any one of [27] to [39], wherein the second organic solvent is the same solvent as the first organic solvent.

[41] The method according to any one of [1] to [40], wherein the step (A) and step (B) are performed in the presence of a first organic solvent, and

wherein the volume of the first organic solvent per g of the mixture to be purified is 1 ml or more and 40 ml or less over the step (A) and the step (B).

[42] The method according to any one of [27] to [41], wherein the volume of the first organic solvent per g of the mixture to be purified at the completion of the step (A) is 1 ml or more and 50 ml or less when the proportion of the second organic solvent is increased in the step (B).

[43] The method according to any one of [27] to [42], wherein the volume of the first organic solvent per g of the mixture to be purified at the completion of the step (A) is 1 ml or more and 100 ml or less when the proportion of the second organic solvent is not increased in the step (B).

[44] The method according to any one of [1] to [43], wherein the steps (A) and (B) are performed in the presence of a second organic solvent, and the volume of the second organic solvent per g of the mixture to be purified is 0 ml or more and 100 ml or less over the step (A) and the step (B).

[45] The method according to any one of [27] to [44], wherein the volume of the second organic solvent per g of the

mixture to be purified at the completion of the step (B) is 0 ml or more and 100 ml or less.

[46] The method according to any one of [34] to [45], wherein the volume of the water per g of the mixture to be purified is 0.1 ml or more and 50 ml or less over the step (A) and the step (B).

[47] The method according to any one of [25] to [46], wherein the volume of the water per g of the mixture to be purified at the completion of the step (A) is 0.1 ml or more and 50 ml or less.

[48] The method according to any one of [34] to [47], wherein the volume of the water per g of the mixture to be purified at the completion of the step (B) is 0.1 ml or more and 50 ml or less.

[49] The method according to any one of [1] to [48], wherein the step (A) is performed in a temperature range of -20°C or higher and 100°C or lower.

[50] The method according to any one of [1] to [49], wherein the step (B) is performed in a temperature range of -20°C or higher and 100°C or lower.

[51] The method according to any one of [1] to [50], wherein the amount of substance of the lithium-containing substance in all liquid components at the completion of the step (A) is 0.5 equivalents or more and 2.0 equivalents or less, based on the amount of substance of the purification target.

[52] The method according to any one of [1] to [51], wherein the lithium-containing substance is at least one selected from the group consisting of lithium hydroxide, lithium carbonate, lithium hydride, trilithium phosphate, lithium methoxide, lithium ethoxide, lithium isopropoxide, lithium tert-butoxide, methyllithium, n-butyllithium, sec-butyllithium, tert-butyllithium, lithium amide, lithium diisopropylamide, lithium hexamethyldisilazide, and lithium tetramethylpiperidide.

[53] The method according to any one of [27] to [52], wherein, in the step (A), the lithium-containing substance, the purification target and the first organic solvent are mixed simultaneously.

[54] The method according to any one of [27] to [53], wherein, in the step (A), a mixed solution of the purification target and the first organic solvent is mixed with the lithium-containing substance.

[55] The method according to any one of [27] to [54], wherein, in the step (A), a mixed solution of the lithium-containing substance and the first organic solvent is mixed with the mixture to be purified.

[56] The method according to any one of [1] to [55], wherein the amino acid or an N-terminal amino acid constituting the peptide compound is represented by the following general formula (1):

[Formula 1]

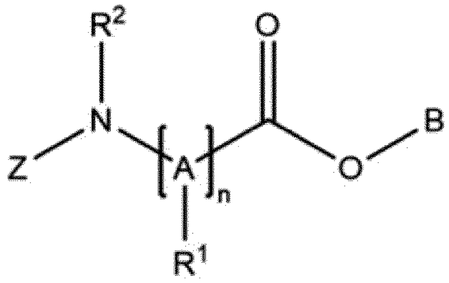

···(1)

wherein

in the general formula (1),
n represents a number of 1 or more and 3 or less,
when a plurality of $R^1$ are present, $R^1$ are the same as or different from each other,
$R^1$ and $R^2$ each represent a hydrogen atom, an alkyl group, an alkenyl group, an alkynyl group, a cycloalkyl group, an aryl group, a heterocyclyl group, or a heteroaryl group, or a substituent in which two or more selected from the group consisting of these substituents are bonded, these substituents optionally have a substituent, each of these groups is optionally a saturated hydrocarbon group or an unsaturated hydrocarbon group,
A represents a carbon atom,
B represents a hydrogen atom or a binding point to an amino acid or a peptide compound,
Z represents a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group,
$R^1$ and $R^2$ are optionally bonded to form a ring together with N and A, wherein each of $R^1$ and $R^2$ is a substituent having a structure obtained by eliminating one hydrogen atom from the structure of the substituent that is not involved in ring formation, and
when n is 2, at least one of $R^2$ and two $R^1$ is not a hydrogen atom.

[57] The method according to any one of [1] to [56], wherein the amino acid or an N-terminal amino acid constituting the peptide compound is at least one selected from the group consisting of (S)-2-(tert-butoxycarbonylamino)-3-phenylpropanoic acid, 3-(tert-butoxycarbonylamino)propanoic acid, 3-(benzyloxycarbonylamino)propanoic acid, (S)-2-(benzyloxycarbonyl(methyl)amino)-2-cyclopentyl-acetic acid, (S)-2-(benzyloxycarbonyl(methyl)amino)-3-phenylpropanoic acid, 3-(9H-fluoren-9-ylmethoxycarbonylamino)propanoic acid, (R)-3-(9H-fluoren-9-ylmethoxycarbonylamino)-3-phenyl-propanoic acid, and (S)-1-(9H-fluoren-9-ylmethoxycarbonyl)pyrrolidine-2-carboxylic acid.
[58] The method according to any one of [1] to [57], wherein the amino acid or an N-terminal amino acid constituting the peptide compound is obtained by a reductive amination reaction catalyzed by a polypeptide comprising an amino acid sequence having 90% or higher identity to an amino acid sequence represented by SEQ ID NO: 1.
[59] The method according to [58], wherein the polypeptide comprises an amino acid sequence represented by SEQ ID NO: 1 with one or more amino acid residues thereof engineered.
[60] The method according to [58] or [59], wherein the polypeptide comprises an amino acid sequence represented by SEQ ID NO: 8.
[61] The method according to any one of [58] to [60], wherein the polypeptide comprises an amino acid sequence other than the sequence having 90% or higher sequence identity to an amino acid sequence represented by SEQ ID NO: 1 with one amino acid residue thereof engineered at any one or both of the N terminus and the C terminus.
[62] The method according to any one of [58] to [61], wherein the polypeptide comprises a tag sequence at any one or both of the N terminus and the C terminus.
[63] The method according to any one of [58] to [62], wherein the polypeptide comprises one or more selected from the group consisting of a streptavidin-bound peptide tag sequence and a His tag sequence at any one or both of the N terminus and the C terminus.
[64] The method according to any one of [58] to [63], wherein the number of amino acid residues of the polypeptide is 300 or more and 400 or less.
[65] The method according to any one of [1] to [64], wherein a purity of the precipitate obtained in the step (B) is 95% by mol or higher.
[66] The method according to any one of [1] to [65], wherein an apparent purity of the precipitate obtained in the step (B) is 95% or higher.
[67] The method according to any one of [1] to [66], wherein a content percentage of impurities in the precipitate obtained in the step (B) is 5% by mol or lower.
[68] The method according to any one of [1] to [67], wherein an apparent content percentage of impurities in the precipitate obtained in the step (B) is 5% or lower.
[69] The method according to any one of [1] to [68], wherein the content percentage of impurities in the mixture to be purified is reduced.
[70] The method according to any one of [1] to [69], wherein the apparent content percentage of impurities in the mixture to be purified is reduced.
[71] The method according to any one of [1] to [70], wherein the salt of the amino acid or the salt of the peptide compound or the solvate thereof is a lithium salt of an amino acid or a lithium salt of a peptide compound or a solvate thereof.
[72] The method according to any one of [1] to [71], further comprising a step of converting the lithium salt into a salt other than the lithium salt.
[73] A method for producing an amino acid or a peptide compound, or a salt thereof, or a solvate thereof, comprising the step according to any one of [1] to [72].
[74] A method for producing a peptide compound, or a salt thereof, or a solvate thereof, comprising the step according to any one of [1] to [73].
[75] A method for producing a cyclic peptide compound, comprising the method according to any one of [1] to [74], and comprising a step of binding the amino acid or the peptide compound, or the solvate thereof, to an amino acid or a peptide compound.
[76] The method according to any one of [73] to [75], comprising a step of cyclizing the peptide compound or the peptide compound obtained by binding.
[77] A lithium salt of an amino acid or a lithium salt of a peptide compound or a solvate thereof, wherein

the lithium salt of an amino acid contains a lithium salt of an amino acid represented by the general formula (1) with a purity of 99% by mol or higher or an apparent purity of 99% or higher, and
the lithium salt of a peptide compound contains a lithium salt of a peptide compound having a residue of an amino acid represented by the general formula (1) with a purity of 99% by mol or higher or an apparent purity of 99%.

[78] The lithium salt of an amino acid or the lithium salt of a peptide compound or the solvate thereof according to [77], wherein the lithium salt is a crystal.

[Advantageous Effect of Invention]

The present invention can provide a method for obtaining a salt of an amino acid or a salt of a peptide compound or a solvate thereof with a high purity.

[Description of Embodiments]

In the present specification, the term "one or more" means a number of 1 or 2 or larger. When the term "one or more" is used in a context related to a substituent for a certain group, this term means a number from 1 to the maximum number of substituents accepted by the group. Examples of the term "one or more" specifically include 1, 2, 3, 4, 5, 6, 7, 8, 9, 10, and/or larger numbers.

In the present specification, the term "to" that indicates a range includes values of both ends thereof. For example, "A to B" means the range of A or more and B or less.

In the present specification, the term "approximately", when used in combination with a numeric value, means the value range of +10% and 10% of the numeric value.

In the present invention, the term "and/or" is meant to include every combination of the terms "and" and "or" appropriately combined. Specifically, for example, the term "A, B and/or C" includes the following seven engineerings: (i) A, (ii) B, (iii) C, (iv) A and B, (v) A and C, (vi) B and C, and (vii) A, B and C.

The embodiments of the present invention will be described. However, the present invention is not limited by embodiments given below.

One embodiment of the present invention is a method for producing a salt of an amino acid or a salt of a peptide compound or a solvate thereof, comprising the following steps (A) and (B):
step (A); a step of contacting a lithium-containing substance with a mixture to be purified which is a mixture of the following purification target (i) and the following impurities (ii):

(i) an amino acid having a protective group at the N terminus or the peptide compound having a protective group at the N terminus, and
(ii) compounds other than the purification target, and step (B); a step of precipitating a lithium salt of the purification target.

An alternative embodiment of the present invention provides a method for removing impurities from a mixture to be purified, comprising the steps (A) and (B).

A further alternative embodiment of the present invention provides a lithium salt of an amino acid or a lithium salt of a peptide compound or a solvate thereof, wherein the lithium salt of an amino acid contains a lithium salt of an amino acid represented by the general formula (1) with a purity of 99% by mol or higher or an apparent purity of 99% or higher, and the lithium salt of a peptide compound contains a lithium salt of a peptide compound having a residue of an amino acid represented by the general formula (1) with a purity of 99% by mol or higher or an apparent purity of 99%.

For conventional purification of the purification target mentioned above, it is difficult to purify the purification target with a high purity. In general, the purification target is used as a starting material for peptide compound and protein synthesis, etc. Obtainment of a peptide compound and a protein with a high purity is important for suppressing adverse reaction ascribable to impurities when the peptide compound and the protein are medicaments. Specifically, purification of the starting material constituting them with a high purity is important. As a result of conducting diligent studies, the present inventors have newly found that, surprisingly, the purification target is converted to lithium salt and can thereby be purified with a high purity. Thus, the present inventors have improved the usefulness of the purification target and its usefulness in peptide compound and protein synthesis using the purification target.

The salt of the amino acid or the salt of the peptide compound or the solvate thereof, more preferably the salt of the amino acid or the salt of the peptide compound, still more preferably the lithium salt of the amino acid or the lithium salt of the peptide compound, according to the present invention is obtained by treating the mixture to be purified by the step (A) and the step (B). In the present invention, the mixture to be purified is a mixture of the purification target and the impurities. In the present specification, the purification target refers to the amino acid having a protective group at the N terminus or the peptide compound having a protective group at the N terminus. Examples of the purification target include an amino acid represented by the general formula (1). In the present specification, the impurities refer to compounds other than the purification target contained in the mixture to be purified. Examples of the impurities include semi-specific impurities (amino acids having a protective group at the N terminus or peptide compounds having a protective group at the N terminus, other than the purification target). More specific examples thereof include β-alanine and derivatives thereof. In the present specification, the derivative refers to, assuming that a certain organic compound is a matrix, a compound engineered by introduction of a functional group, oxidation, reduction, replacement of an atom, or the like to the extent that the structure or properties of the matrix are not drastically changed. Examples of the derivative of β-alanine include specific

impurities (β-alanine having a protective group at the N terminus).

In the present specification, the "amino acid" includes a natural amino acid and a non-natural amino acid (also referred to as an amino acid derivative). Also in the present specification, the "amino acid residue" includes a natural amino acid residue and a non-natural amino acid (amino acid derivative) residue.

The natural amino acid refers to glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro).

Examples of the non-natural amino acid (amino acid derivative) include, but are not particularly limited to, β-amino acids, D-amino acids, N-substituted amino acids (excluding Pro), α,α-disubstituted amino acids, amino acids having a side chain different from the natural one, and hydroxycarboxylic acid. In the present specification, the non-natural N-substituted amino acid means a N-substituted amino acid other than Pro.

**In** the present specification, the amino acid accepts an arbitrary conformation. The side chain of the amino acid can be selected without particular limitations and is freely selected from a hydrogen atom as well as, for example, an alkyl group, an alkenyl group, an alkynyl group, an aryl group, a heteroaryl group, an aralkyl group, a heteroaralkyl group, a cycloalkyl group, and a spiro-bonded cycloalkyl group. A substituent may be added to each of the groups. Such a substituent is not limited and can be one or two or more substituents each independently freely selected from arbitrary substituents including a halogen atom, an O atom, a S atom, a N atom, a B atom, a Si atom, and a P atom. Specifically, examples thereof include an optionally substituted alkyl group, alkoxy group, alkenyl group, alkynyl group, aryl group, heteroaryl group, aralkyl group, cycloalkyl group, and the like, or oxo, aminocarbonyl, and a halogen atom. The amino acid according to one embodiment may be a compound having a carboxy group and an amino group in the same molecule (even in this case, the amino acid also includes imino acids such as proline and hydroxyproline).

The step (A) is the step of contacting a lithium-containing substance with the mixture to be purified. The step (A) may form lithium salt of the purification target and may form lithium salt of the impurities. The step (B) is the step of precipitating lithium salt of the purification target. A portion of the step (A) and a portion of the step (B) may be performed such that their times overlap with each other. Before the step (A), the mixture to be purified may be purified by a method other than the method of the present invention. For example, the purification target may be precipitated in a free form and purified, without forming lithium salt of the purification target. At least one of the step (A) and the step (B) may be performed in the presence of a solvent. **In** the present specification, generation of a solid from a solvent refers to "precipitation".

The protective group of the purification target may be a carbamate group. The carbamate group refers to a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group and may be a fluorenylmethoxycarbonyl group, a tert-butoxycarbonyl group, or a benzyloxycarbonyl group, may be a fluorenylmethoxycarbonyl group or a benzyloxycarbonyl group, may be a fluorenylmethoxycarbonyl group, or may be a benzyloxycarbonyl group.

The protective group of the impurities may be a carbamate group. The carbamate group refers to a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group and may be a fluorenylmethoxycarbonyl group, a tert-butoxycarbonyl group, or a benzyloxycarbonyl group, may be a fluorenylmethoxycarbonyl group or a benzyloxycarbonyl group, may be a fluorenylmethoxycarbonyl group, or may be a benzyloxycarbonyl group. These protective groups may be protective groups that protect the N terminus of the impurities.

The protective group of the purification target and the protective group of the impurities may be different or may be the same. The protective group of the purification target and the protective group of the impurities are preferably the same.

The step (A) may be performed in the presence of a first organic solvent. The first organic solvent is a solvent that can dissolve the purification target from the viewpoint of allowing the contact of the lithium-containing substance with the purification target to proceed efficiently. The solubility of the purification target in the first organic solvent is preferably 20 g/L or more. The solubility of the lithium salt of the purification target in the first organic solvent is preferably 20 g/L or less. The solubility of the impurities in the first organic solvent is preferably 20 g/L or more.

The first organic solvent is, for example, at least one selected from the group consisting of nitriles such as acetonitrile, alcohols such as methanol, ethanol, 1-propanol, 2-propanol, 1-butanol, 2-butanol, 2-methyl-2-propanol, and ethylene glycol, ethers such as tetrahydrofuran, 1,4-dioxane, 2-methyltetrahydrofuran, methyl tert-butyl ether, diisopropyl ether, diethyl ether, and 1,2-dimethoxyethane, ketones such as acetone, methyl ethyl ketone, and methyl isobutyl ketone, amides such as dimethylformamide, dimethylacetamide, and N-methyl-2-pyrrolidone, ureas such as 1,3-dimethyl-2-imidazolidinone, sulfoxides such as dimethyl sulfoxide, sulfones such as sulfolane, alkanes such as heptane, methylcyclohexane, hexane, cyclohexane, and pentane, aromatic compounds such as benzene, toluene, 1,2-dimethylbenzene, 1,3-dimethylbenzene, and 1,4-dimethylbenzene, esters such as ethyl acetate and isopropyl ester, and alkyl halides such as dichloroethane, dichloromethane, chloroform, and carbon tetrachloride. The first solvent may be of plural types and is preferably of one type from the viewpoint of the simplified step (A).

The step (A) may be performed in the presence of water.

The step (B) may be performed in the presence of a second organic solvent. The second organic solvent is a solvent in which the solubility of the lithium salt of the purification target is small, i.e., a solvent that functions as a poor solvent for the lithium salt of the purification target. The solubility of the lithium salt of the purification target in the second organic solvent is preferably 10 g/L or less.

The second organic solvent is, for example, at least one selected from the group consisting of nitriles such as acetonitrile, alcohols such as methanol, ethanol, 1-propanol, 2-propanol, 1-butanol, 2-butanol, 2-methyl-2-propanol, and ethylene glycol, ethers such as tetrahydrofuran, 1,4-dioxane, 2-methyltetrahydrofuran, methyl tert-butyl ether, diisopropyl ether, diethyl ether, and 1,2-dimethoxyethane, ketones such as acetone, methyl ethyl ketone, and methyl isobutyl ketone, amides such as dimethylformamide, dimethylacetamide, and N-methyl-2-pyrrolidone, ureas such as 1,3-dimethyl-2-imidazolidinone, sulfoxides such as dimethyl sulfoxide, sulfones such as sulfolane, alkanes such as heptane, methylcyclohexane, hexane, cyclohexane, and pentane, aromatic compounds such as benzene, toluene, 1,2-dimethylbenzene, 1,3-dimethylbenzene, and 1,4-dimethylbenzene, esters such as ethyl acetate and isopropyl ester, and alkyl halides such as dichloroethane, dichloromethane, chloroform, and carbon tetrachloride. The second solvent may be of plural types and is preferably of one type from the viewpoint of the simplified step (B).

The step (B) may be performed in the presence of water and is preferably performed in the presence of water from the viewpoint of dissolving lithium salt of the impurities. The solubility of the lithium salt of the impurities in water is preferably 10 g/L or more.

The second organic solvent may be used with the first organic solvent in the step (A).

In the step (B), lithium salt of the purification target is precipitated. The precipitation of the lithium salt of the purification target may be promoted by adding the second organic solvent upon precipitation and increasing its proportion. In the case of increasing the proportion of the second organic solvent, the second organic solvent may be added to the first organic solvent used in the step (A).

The proportion of the first organic solvent or the proportion of the second organic solvent means a volume/mass ratio (ml/g; hereinafter, also referred to as v/w) calculated by dividing the volume of the first organic solvent or the second organic solvent by the mass of the purification target.

When the lithium salt of the purification target has sufficiently small solubility in the first organic solvent used in the step (A), the first organic solvent and the second organic solvent may be the same solvent. In this case, it is not necessary to add the second organic solvent in the step (B). In this case, the step (B) starts immediately after contact of the lithium-containing substance with the purification target in the step (A) so that lithium salt of the purification target is precipitated.

The lithium salt of the purification target is preferably precipitated as a solid. In this context, the solid includes crystals or an amorphous form. The lithium salt is more preferably precipitated as crystals (crystallized). In this case, the step (B) may be regarded as the step of crystallizing lithium salt of the purification target.

The volume of the first organic solvent per g of the mixture to be purified may be 1 ml or more, 2 ml or more, or 3 ml or more and may be 100 ml or less, 80 ml or less, 60 ml or less, or 40 ml or less, over the step (A) and the step (B). The volume of the first organic solvent per g of the mixture to be purified may be between 1 ml and 100 ml, between 1 ml and 80 ml, between 2 ml and 60 ml, or between 3 ml and 40 ml over the step (A) and the step (B).

The volume of the first organic solvent per g of the mixture to be purified at the completion of the step (A) (initial concentration of the first organic solvent) may be 1 ml or more, 2 ml or more, or 3 ml or more and may be 50 ml or less, 40 ml or less, 30 ml or less, or 20 ml or less when the proportion of the second organic solvent is increased in the step (B). The initial concentration of the first organic solvent may be between 1 ml and 50 ml, between 1 ml and 40 ml, between 2 ml and 30 ml, or between 3 ml and 20 ml.

The volume of the first organic solvent per g of the mixture to be purified at the completion of the step (A) (initial concentration of the first organic solvent) may be 1 ml or more, 10 ml or more, 20 ml or more, 30 ml or more, or 35 ml or more and may be 100 ml or less, 75 ml or less, 50 ml or less, or 37 ml or less when the proportion of the second organic solvent is not increased in the step (B). The initial concentration of the first organic solvent may be between 1 ml and 100 ml, between 10 ml and 75 ml, between 20 ml and 50 ml, between 30 ml and 37 ml, or between 35 ml and 37 ml.

The volume of the second organic solvent per g of the mixture to be purified may be 0 ml or more and may be 40 ml or less, 60 ml or less, 80 ml or less, or 100 ml or less, over the steps (A) and (B). The volume of the second organic solvent per g of the mixture to be purified may be between 0 ml and 100 ml, between 0 ml and 80 ml, between 1 ml and 60 ml, or between 1 ml and 40 ml, over the steps (A) and (B).

The volume of the second organic solvent per g of the mixture to be purified at the completion of the step (B) may be 0 ml or more and may be 40 ml or less, 60 ml or less, 80 ml or less, or 100 ml or less. The volume of the second organic solvent per g of the mixture to be purified at the completion of the step (B) may be between 0 ml and 100 ml, between 0 ml and 80 ml, between 0 ml and 60 ml, or between 0 ml and 40 ml.

The volume of the water per g of the mixture to be purified may be 0.1 ml or more, 0.3 ml or more, or 0.5 ml or more and may be 50 ml or less, 30 ml or less, 10 ml or less, or 5 ml or less, over the steps (A) and (B). The volume of the water in the solvents may be between 0.1 ml and 50 ml, between 0.1 ml and 30 ml, between 0.3 ml and 10 ml, or between 0.5 ml and 5 ml, over the steps (A) and (B).

The volume of the water per g of the mixture to be purified at the completion of the step (A) (initial concentration of the water) may be 0.1 ml or more, 0.3 ml or more, or 0.5 ml or more and may be 50 ml or less, 30 ml or less, 10 ml or less, or 4 ml or less. The initial concentration of the water may be between 0.1 ml and 50 ml, between 0.1 ml and 30 ml, between 0.3 ml and 10 ml, or between 0.5 ml and 4 ml.

The volume of the water per g of the mixture to be purified at the completion of the step (B) (initial concentration of the water) may be 0.1 ml or more, 0.3 ml or more, or 0.5 ml or more and may be 50 ml or less, 30 ml or less, 10 ml or less, or 4 ml or less. The initial concentration of the water may be between 0.1 ml and 50 ml, between 0.1 ml and 30 ml, between 0.3 ml and 10 ml, or between 0.5 ml and 4 ml.

The step (A) and the step (B) may be performed in the presence of an additional solvent other than the first organic solvent and the second organic solvent. Examples of the additional solvent include buffer solutions such as phosphate buffer solutions, CHES (N-cyclohexyl-2-aminoethanesulfonic acid), Tris (trishydroxymethylaminomethane), and Bicine (N,N-di(2-hydroxyethyl)glycine).

The temperature at which the step (A) is performed may be -20°C or higher, may be 0°C or higher, may be 10°C or higher, or may be 20°C or higher. The temperature at which the step (A) is performed may be 100°C or lower, may be 80°C or lower, may be 60°C or lower, may be 40°C or lower, or may be 30°C or lower. The temperature at which the step (A) is performed may be -20 to 100°C, may be 0 to 80°C, may be 10 to 60°C, may be 10 to 40°C, or may be 20 to 30°C.

The temperature at which the step (B) is performed may be -20°C or higher, may be 0°C or higher, may be 10°C or higher, may be 20°C or higher, may be 30°C or higher, or may be 35°C or higher. The temperature at which the step (A) is performed may be 100°C or lower, may be 80°C or lower, may be 60°C or lower, may be 40°C or lower, or may be 30°C or lower. The temperature at which the step (A) is performed may be -20 to 100°C, may be 0 to 80°C, may be 10 to 60°C, may be 10 to 40°C, or may be 20 to 30°C.

The conditions, such as temperature and concentrations of substances contained in various solutions, of the step (A) and the step (B) may be the same or may be different. When these conditions are different between these steps, conditions selected from among various conditions of the step (A) mentioned above may be combined.

The amount of substance of the lithium-containing substance at the completion of the step (A) may be 0.5 equivalents or more, 0.8 equivalents or more, or 1.0 equivalent or more and may be 2.0 equivalents or less, 1.5 equivalents or less, or 1.1 equivalents or less, based on the amount of substance of the purification target. The amount of the lithium-containing substance based on the mass of the reactant may be 0.5 equivalents or more and 2.0 equivalents or less, 0.8 equivalents or more and 1.5 equivalents or less, or 1.0 equivalent or more and 1.1 equivalents or less.

In the present specification, the lithium-containing substance refers to a substance that contains lithium for contact with the purification target in the step (A). The lithium-containing substance for use in the step (A) is, for example, at least one selected from the group consisting of lithium hydroxide, lithium tert-butoxide, lithium hydroxide, lithium carbonate, lithium hydride, trilithium phosphate, lithium methoxide, lithium ethoxide, lithium isopropoxide, lithium tert-butoxide, methyllithium, n-butyllithium, sec-butyllithium, tert-butyllithium, lithium amide, lithium diisopropylamide, lithium hexamethyldisilazide, and lithium tetramethylpiperidide.

The method for contacting the lithium-containing substance with the mixture to be purified is not limited, and the mixture to be purified and the lithium-containing substance may be contacted with each other in the presence of the first organic solvent. In this case, the mixture to be purified, the lithium-containing substance, and the first organic solvent may be mixed in the same container from the beginning; the mixture to be purified and the first organic solvent may be mixed, and the lithium-containing substance can be added to the obtained mixed solution; or the lithium-containing substance and the first organic solvent may be mixed, and the mixture to be purified may be added to the obtained mixed solution.

The content of the purification target in the mixture to be purified, based on which the amounts of the first organic solvent, the second organic solvent, and the lithium-containing substance are determined may be an actually measured value, an estimated value, or a calculated value and is preferably an actually measured value or a calculated value, most preferably an actually measured value.

Examples of the amino acid having a protective group at the N terminus or an amino acid constituting a peptide compound having a protective group at the N terminus, included in the purification target include an amino acid represented by the general formula (1) (also referred to as an amino acid (1)), hydrophobic amino acids, aliphatic amino acids, and aromatic amino acids.

n represents a number of 1 or more and 3 or less and may be an integer of 1 or more and 3 or less. n may be 1, 2, or 3. When n is 2, at least one of $R^2$ and two $R^1$ is not a hydrogen atom, two or more of $R^2$ and two $R^1$ may not be a hydrogen atom, and all three of $R^2$ and two $R^1$ may not be a hydrogen atom.

Each of $R^1$ and $R^2$ represents a hydrogen atom, an alkyl group, an alkenyl group, an alkynyl group, a cycloalkyl group, an aryl group, a heterocyclyl group, or a heteroaryl group, or a substituent in which two or more selected from the group consisting of these substituents are bonded (e.g., a benzyl group), these substituents optionally have a substituent, and these groups may be saturated or unsaturated.

A represents a carbon atom, and B represents a hydrogen atom or a binding point to an amino acid or a peptide compound.

Z represents a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group.

$R^1$ and $R^2$ are optionally bonded to form a ring together with N and A. In this case, each of $R^1$ and $R^2$ is a substituent having a structure obtained by eliminating one hydrogen atom from the structure of the substituent that is not involved in ring formation.

In the present specification, the "alkyl" is a monovalent group induced by the removal of one arbitrary hydrogen atom from aliphatic hydrocarbon, and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear form but a branched form. The alkyl is specifically an alkyl group having 1 to 20 carbon atoms ($C_1$ to $C_{20}$; hereinafter, "$C_p$ to $C_q$" means that the number of carbon atoms is p to q), preferably a $C_1$ to $C_{10}$ alkyl, more preferably a $C_1$ to $C_6$ alkyl. Examples of the alkyl specifically include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

In the present specification, the "alkylene" means a divalent group induced by the further removal of one arbitrary hydrogen atom from the "alkyl" described above, and is preferably $C_4$ to $C_8$ alkylene. Examples of the alkylene specifically include $-CH_2-$, $-(CH_2)_2-$, $-(CH_2)_3-$, $-CH(CH_3)CH_2-$, $-C(CH_3)_2-$, $-(CH_2)_4-$, $-CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$, $-(CH_2)_5-$, $-(CH_2)_6-$, $-(CH_2)_7-$, $-(CH_2)_8-$.

In the present specification, the "alkenyl" is a monovalent group having at least one double bond (two adjacent $SP^2$ carbon atoms). Depending on the conformation of the double bond and a substituent (if present), the geometric morphology of the double bond can assume entgegen (E) or zusammen (Z) and cis or trans conformations. The alkenyl includes not only a linear form but a branched form. The alkenyl is preferably $C_2$ to $C_{10}$ alkenyl, more preferably $C_2$ to $C_6$ alkenyl. Examples thereof specifically include vinyl, allyl, 1-propenyl, 2-propenyl, 1-butenyl, 2-butenyl (which includes cis and trans), 3-butenyl, pentenyl, 3-methyl-2-butenyl, and hexenyl.

In the present specification, the "alkynyl" is a monovalent group having at least one triple bond (two adjacent SP carbon atoms). The alkynyl includes not only a linear form but a branched form. The alkynyl is preferably $C_2$ to $C_{10}$ alkynyl, more preferably $C_2$ to $C_6$ alkynyl. Examples thereof specifically include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

In the present specification, the "cycloalkyl" means a saturated or partially saturated cyclic monovalent aliphatic hydrocarbon group and includes a monocyclic ring, a bicyclo ring, and a spiro ring. The cycloalkyl is preferably $C_3$ to $C_8$ cycloalkyl. Examples thereof specifically include cyclopropyl, cyclobutyl, cyclopentyl, cyclohexyl, cycloheptyl, cyclooctyl, bicyclo[2.2.1]heptyl, and spiro[3.3]heptyl.

In the present specification, the "aryl" means a monovalent aromatic hydrocarbon ring and is preferably $C_6$ to $C_{10}$ aryl. Examples of the aryl specifically include phenyl and naphthyl (e.g., 1-naphthyl and 2-naphthyl).

In the present specification, the "heterocyclyl" means a nonaromatic cyclic monovalent group containing a carbon atom as well as 1 to 5 heteroatoms. The heterocyclyl may have a double and/or triple bond in the ring. A carbon atom in the ring may form carbonyl through oxidation, and the ring may be a monocyclic ring or a condensed ring. The number of atoms constituting the ring is preferably 4 to 10 (4- to 10-membered heterocyclyl), more preferably 4 to 7 (4- to 7-membered heterocyclyl). Examples of the heterocyclyl specifically include azetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, azetidinyl, oxazolidone, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

In the present specification, the "heteroaryl" means an aromatic cyclic monovalent group containing a carbon atom as well as 1 to 5 heteroatoms. The ring may be a monocyclic ring or a condensed ring with another ring and may be partially saturated. The number of atoms constituting the ring may be 5 to 12 (5- to 12-membered heteroaryl), may be 6 to 10 (5- to 12-membered heteroaryl), or may be 6 or 7 (6- or 7-membered heteroaryl). Examples of the heteroaryl specifically include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

As used herein, "optionally substituted" means "may have a substituent", meaning that a group may be substituted by an arbitrary substituent. Examples of the substituent include a halogen atom, an oxygen atom, a sulfur atom, a nitrogen atom, a boron atom, a silicon atom, and a phosphorus atom. More specific examples of the substituent

include an alkyl group, an alkoxy group, a fluoroalkyl group, a fluoroalkoxy group, an oxo group, an aminocarbonyl group, an alkylsulfonyl group, an alkylsulfonylamino group, a cycloalkyl group, an aryl group, a heteroaryl group, a heterocyclyl group, an arylalkyl group, a heteroarylalkyl group, a halogen group, a nitro group, an amino group, a monoalkylamino group, a dialkylamino group, a cyano group, a carboxyl group, an alkoxycarbonyl group, and a formyl group.

The amino acid or an N-terminal amino acid constituting the peptide compound can be obtained by a reductive amination reaction catalyzed by a polypeptide comprising an amino acid sequence having 90% or higher identity to the amino acid sequence represented by SEQ ID NO: 1.

The identity to the amino acid sequence represented by SEQ ID NO: 1 may be 90% or higher, 91% or higher, 92% or higher, 93% or higher, 94% or higher, 95% or higher, 96% or higher, 97% or higher, 98% or higher, 99% or higher or 100%.

The identity to the amino acid sequence represented by SEQ ID NO: 1 can be determined with algorithm BLAST by Karlin and Altschul (Proc. Natl. Acad. Sci. USA (1993) 90: 5873-7). A program called BLASTN or BLASTX has been developed on the basis of this algorithm (Altschul et al., J. Mol. Biol. (1990) 215: 403-10). In the case of analyzing an amino acid sequence with BLASTX on the basis of BLAST, the parameters are set to Score = 50 and Wordlength = 3. In the case of using BLAST and Gapped BLAST programs, the default parameters of each program are used. For specific approaches of these analysis methods, see information on the website of BLAST (basic local alignment search tool) of NCBI (Natianal Center for Biotechnology Information) known in the art.

The polypeptide according to the present embodiment may be a polypeptide having the engineering of one or more amino acid residues in the amino acid sequence represented by SEQ ID NO: 1, and 1 to 20, 1 to 15, 1 to 10, 1 to 7 or 1 to 5 amino acid residues may be engineered. The number of engineered amino acid residues may be 3 or less, may be 2 or less, or may be 1.

The engineering may be one or more selected from the group consisting of substitution, deletion and insertion and can be substitution. The engineering may be conservative engineering. The conservative engineering means the engineering of an amino acid residue so as not to reduce the catalytic activity of interest as compared with the polypeptide before the engineering.

The engineering may be substitution by a natural amino acid different from the amino acid residue before the engineering. The natural amino acid for use in the substitution may be one or more selected from the group consisting of glycine, alanine, serine, threonine, valine, leucine, isoleucine, phenylalanine, tyrosine, tryptophan, histidine, glutamine, asparagine, glutamic acid, aspartic acid, cysteine, methionine, lysine, arginine and proline.

The polypeptide according to the present embodiment may be a polypeptide comprising a sequence having the engineering of an amino acid residue positioned at a site corresponding to at least one amino acid residue selected from the group consisting of a histidine residue at position 44, a phenylalanine residue at position 117, a methionine residue at position 141, a threonine residue at position 156, a histidine residue at position 182, a glutamine residue at position 186, a tryptophan residue at position 253, and a lysine residue at position 260 in the amino acid sequence represented by SEQ ID NO: 1, may be a polypeptide comprising a sequence having the engineering of an amino acid residue positioned at a site corresponding to at least one amino acid residue selected from the group consisting of a methionine residue at position 141, a histidine residue at position 182, a tryptophan residue at position 253, and a lysine residue at position 260 because the catalytic activity for reductive amination reaction is further improved, and may be a polypeptide comprising a sequence having the engineering of an amino acid residue positioned at a site corresponding to at least one amino acid residue selected from the group consisting of a methionine residue at position 141, a tryptophan residue at position 253, and a lysine residue at position 260.

The polypeptide according to the present embodiment may be a polypeptide comprising a sequence having the substitution of an amino acid residue positioned at a site corresponding to a histidine residue at position 44 in the amino acid sequence represented by SEQ ID NO: 1 by an amino acid residue other than histidine residue. The polypeptide may be a polypeptide comprising the amino acid sequence represented by SEQ ID NO: 2. The amino acid residue represented by X in SEQ ID NO: 2 is an alanine residue, an aspartic acid residue, a glutamic acid residue, a phenylalanine residue, a glycine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a histidine residue at position 44 by a methionine residue because the catalytic activity for reductive amination reaction is further improved. Specifically, the polypeptide according to the present embodiment may comprise an amino acid sequence (H44M) having the substitution of an amino acid residue positioned at a site corresponding to a histidine residue at position 44 in the amino acid sequence represented by SEQ ID NO: 1 by a methionine residue.

The polypeptide according to the present embodiment may be a polypeptide comprising a sequence having the substitution of an amino acid residue positioned at a site corresponding to a phenylalanine residue at position 117 in the amino acid sequence represented by SEQ ID NO: 1 by an amino acid residue other than a phenylalanine residue. The polypeptide may be a polypeptide comprising the amino acid sequence represented by SEQ ID NO: 3. The amino acid

residue represented by X in SEQ ID NO: 3 is an alanine residue, an aspartic acid residue, a glutamic acid residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a phenylalanine residue at position 117 by a leucine residue because the catalytic activity for reductive amination reaction is further improved. Specifically, the polypeptide according to the present embodiment may comprise an amino acid sequence (F 117L) having the substitution of an amino acid residue positioned at a site corresponding to a phenylalanine residue at position 117 in the amino acid sequence represented by SEQ ID NO: 1 by a leucine residue.

The polypeptide according to the present embodiment may be a polypeptide comprising a sequence having the substitution of an amino acid residue positioned at a site corresponding to a methionine residue at position 141 in the amino acid sequence represented by SEQ ID NO: 1 by an amino acid residue other than methionine residue. The polypeptide may be a polypeptide comprising the amino acid sequence represented by SEQ ID NO: 4. The amino acid residue represented by X in SEQ ID NO: 4 is an alanine residue, an aspartic acid residue, a glutamic acid residue, a phenylalanine residue, a glycine residue, a histidine residue, an isoleucine residue, a lysine residue, a leucine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a methionine residue at position 141 by at least one amino acid residue selected from the group consisting of a tyrosine residue, a tryptophan residue, a valine residue, a threonine residue, a serine residue, an arginine residue, a leucine residue, a lysine residue, an isoleucine residue, a histidine residue, a phenylalanine residue and an alanine residue because the catalytic activity for reductive amination reaction is further improved. Specifically, the polypeptide according to the present embodiment may comprise an amino acid sequence having the substitution of an amino acid residue positioned at a site corresponding to a methionine residue at position 141 in the amino acid sequence represented by SEQ ID NO: 1 by a tyrosine residue, a tryptophan residue, a valine residue, a threonine residue, a serine residue, an arginine residue, a leucine residue, a lysine residue, an isoleucine residue, a histidine residue, a phenylalanine residue, or an alanine residue (M141Y, M141W, M141V, M141T, M141S, M141R, M141L, M141K, M141I, M141H, M141F, or M141A). The polypeptide may comprise a sequence having the substitution of an amino acid residue positioned at a site corresponding to a methionine residue at position 141 by at least one amino acid residue selected from the group consisting of a tyrosine residue, a tryptophan residue, a valine residue, a lysine residue, an isoleucine residue, a phenylalanine residue and an alanine residue. Specifically, the polypeptide according to the present embodiment may comprise an amino acid sequence having the substitution of an amino acid residue positioned at a site corresponding to a methionine residue at position 141 in the amino acid sequence represented by SEQ ID NO: 1 by a tyrosine residue, a tryptophan residue, a valine residue, a lysine residue, an isoleucine residue, a histidine residue, a phenylalanine residue, or an alanine residue (M141Y, M141W, M141V, M141K, M141I, M141H, M141F, or M141A).

The polypeptide according to the present embodiment may be a polypeptide comprising a sequence having the substitution of an amino acid residue positioned at a site corresponding to a threonine residue at position 156 in the amino acid sequence represented by SEQ ID NO: 1 by an amino acid residue other than a threonine residue. The polypeptide may be a polypeptide comprising the amino acid sequence represented by SEQ ID NO: 5. The amino acid residue represented by X in SEQ ID NO: 5 is an alanine residue, an aspartic acid residue, a glutamic acid residue, a phenylalanine residue, a glycine residue, a histidine residue, an isoleucine residue, a lysine residue, a leucine residue, an asparagine residue, a proline residue, a glutamine residue, an arginine residue, a serine residue, a threonine residue, a valine residue, a tryptophan residue, or a tyrosine residue.

The polypeptide can comprise a sequence having the substitution of an amino acid residue positioned at a site corresponding to a threonine residue at position 156 by a serine residue because the catalytic activity for reductive amination reaction is further improved. Specifically, the polypeptide according to the present embodiment can comprise an amino acid sequence having the substitution of an amino acid residue positioned at a site corresponding to a threonine residue at position 156 in the amino acid sequence represented by SEQ ID NO: 1 by a serine residue (T156S).

The polypeptide according to the present embodiment may be a polypeptide comprising a sequence having the substitution of an amino acid residue positioned at a site corresponding to a histidine residue at position 182 in the amino acid sequence represented by SEQ ID NO: 1 by an amino acid residue other than histidine residue. The polypeptide may be a polypeptide comprising the amino acid sequence represented by SEQ ID NO: 6. The amino acid residue represented by X in SEQ ID NO: 6 is an alanine residue, an aspartic acid residue, a glutamic acid residue, a phenylalanine residue, a glycine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a histidine residue at position 182 by at least one amino acid residue selected from the group consisting of

a tyrosine residue, a glutamine residue, a methionine residue, a leucine residue, a glycine residue, a phenylalanine residue and an alanine residue because the catalytic activity for reductive amination reaction is further improved. Specifically, the polypeptide according to the present embodiment may comprise an amino acid sequence having the substitution of an amino acid residue positioned at a site corresponding to a histidine residue at position 182 in the amino acid sequence represented by SEQ ID NO: 1 by a tyrosine residue, a glutamine residue, a methionine residue, a leucine residue, a glycine residue, a phenylalanine residue, or an alanine residue (H182Y, H182Q, H182M, H182L, H182G, H182F, or H182A). The polypeptide may comprise a sequence having the substitution of an amino acid residue positioned at a site corresponding to a histidine residue at position 182 by at least one amino acid residue selected from the group consisting of a methionine residue, a leucine residue, and a phenylalanine residue. Specifically, the polypeptide according to the present embodiment may comprise an amino acid sequence having the substitution of an amino acid residue positioned at a site corresponding to a histidine residue at position 182 in the amino acid sequence represented by SEQ ID NO: 1 by a methionine residue, a leucine residue, or a phenylalanine residue (H182M, H182L, or H182F).

The polypeptide according to the present embodiment may be a polypeptide comprising a sequence having the substitution of an amino acid residue positioned at a site corresponding to a glutamine residue at position 186 in the amino acid sequence represented by SEQ ID NO: 1 by an amino acid residue other than a glutamine residue. The polypeptide may be a polypeptide comprising the amino acid sequence represented by SEQ ID NO: 7. The amino acid residue represented by X in SEQ ID NO: 7 is an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, an arginine residue, a serine residue, a threonine residue, a valine residue, a tryptophan residue, or a tyrosine residue.

The polypeptide may comprise a sequence having the substitution of an amino acid residue positioned at a site corresponding to a glutamine residue at position 186 in the amino acid sequence represented by SEQ ID NO: 1 by at least one amino acid residue selected from the group consisting of a methionine residue and a glutamic acid residue because the catalytic activity for reductive amination reaction is further improved. Specifically, the polypeptide according to the present embodiment may comprise an amino acid sequence having the substitution of an amino acid residue positioned at a site corresponding to a glutamine residue at position 186 in the amino acid sequence represented by SEQ ID NO: 1 by a methionine residue or a glutamic acid residue (Q186M, or Q186E).

The polypeptide according to the present embodiment may be a polypeptide comprising a sequence having the substitution of an amino acid residue positioned at a site corresponding to a tryptophan residue at position 253 in the amino acid sequence represented by SEQ ID NO: 1 by an amino acid residue other than a tryptophan residue. The polypeptide may be a polypeptide comprising the amino acid sequence represented by SEQ ID NO: 8. The amino acid residue represented by X in SEQ ID NO: 8 is an alanine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, or a tyrosine residue.

The polypeptide may comprise a sequence having the substitution of an amino acid residue positioned at a site corresponding to a tryptophan residue at position 253 by at least one amino acid residue selected from the group consisting of a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue and an alanine residue because the catalytic activity for reductive amination reaction is further improved. Specifically, the polypeptide according to the present embodiment can comprise an amino acid sequence having the substitution of an amino acid residue positioned at a site corresponding to a tryptophan residue at position 253 in the amino acid sequence represented by SEQ ID NO: 1 by a tyrosine residue, a valine residue, a threonine residue, a serine residue, an arginine residue, a glutamine residue, a proline residue, an asparagine residue, a methionine residue, a leucine residue, a lysine residue, an isoleucine residue, a histidine residue, a phenylalanine residue, or an alanine residue (W253Y, W253V, W253T, W253S, W253R, W253Q, W253P, W253N, W253M, W253L, W253K, W253I, W253H, W253F, or W253A). The polypeptide may comprise a sequence having the substitution of an amino acid residue positioned at a site corresponding to a tryptophan residue at position 253 by at least one amino acid residue selected from the group consisting of a leucine residue, an isoleucine residue and a histidine residue. Specifically, the polypeptide according to the present embodiment may comprise an amino acid sequence having the substitution of an amino acid residue positioned at a site corresponding to a tryptophan residue at position 253 in the amino acid sequence represented by SEQ ID NO: 1 by a leucine residue, an isoleucine residue or a histidine residue (W253L, W253I, or W253H).

The polypeptide according to the present embodiment may be a polypeptide comprising a sequence having the substitution of an amino acid residue positioned at a site corresponding to a lysine residue at position 260 in the amino acid sequence represented by SEQ ID NO: 1 by an amino acid residue other than a lysine residue. The polypeptide may be a polypeptide comprising the amino acid sequence represented by SEQ ID NO: 9. The amino acid residue represented by X in SEQ ID NO: 9 is an alanine residue, an aspartic acid residue, a glutamic acid residue, a phenylalanine residue, a glycine

residue, a histidine residue, an isoleucine residue, a leucine residue, a methionine residue, an asparagine residue, a proline residue, a glutamine residue, an arginine residue, a serine residue, a threonine residue, a valine residue, a tryptophan residue, or a tyrosine residue.

The polypeptide may comprise a sequence having the substitution of an amino acid residue positioned at a site corresponding to a lysine residue at position 260 by at least one amino acid residue selected from the group consisting of a tyrosine residue, a tryptophan residue, a threonine residue, a serine residue, an arginine residue, a glutamine residue, an asparagine residue, a methionine residue, a leucine residue, a histidine residue, a glycine residue, a phenylalanine residue, a glutamic acid residue and an alanine residue because the catalytic activity for reductive amino reaction is further improved. Specifically, the polypeptide according to the present embodiment may comprise an amino acid sequence having the substitution of an amino acid residue positioned at a site corresponding to a lysine residue at position 260 in the amino acid sequence represented by SEQ ID NO: 1 by a tyrosine residue, a tryptophan residue, a threonine residue, a serine residue, an arginine residue, a glutamine residue, a asparagine residue, a methionine residue, a leucine residue, a histidine residue, a glycine residue, a phenylalanine residue, a glutamic acid residue or an alanine residue (K260Y, K260W, K260T, K260S, K260R, K260Q, K260N, K260M, K260L, K260H, K260G, K260F, K260E, or K260A). The polypeptide may further comprise a sequence having the substitution of an amino acid residue positioned at a site corresponding to a lysine residue at position 260 by at least one amino acid residue selected from the group consisting of a glutamine residue, a methionine residue, a glutamic acid residue and an asparagine residue. Specifically, the polypeptide according to the present embodiment may comprise an amino acid sequence having the substitution of an amino acid residue positioned at a site corresponding to a lysine residue at position 260 in the amino acid sequence represented by SEQ ID NO: 1 by a glutamine residue, a methionine residue, a glutamic acid residue or an asparagine residue (K260Q, K260M, K260E, or K260N).

The polypeptide according to the present embodiment may comprise amino acid sequence a1 represented by SEQ ID NO: 4 wherein the amino acid residue represented by X is a valine residue (mutation: M141V), amino acid sequence a2 represented by SEQ ID NO: 4 wherein the amino acid residue represented by X is a tyrosine residue (mutation: M141Y), amino acid sequence a3 represented by SEQ ID NO: 6 wherein the amino acid residue represented by X is a leucine residue (mutation: H182L), amino acid sequence a4 represented by SEQ ID NO: 8 wherein the amino acid residue represented by X is a histidine residue (mutation: W253H), or amino acid sequence a5 represented by SEQ ID NO: 9 wherein the amino acid residue represented by X is a glutamic acid residue (mutation: K260E).

The polypeptide according to the present embodiment may comprise a sequence having 90% or higher sequence identity to the amino acid sequence a1, a2, a3, a4, or a5. The sequence identity may be 91% or higher, 92% or higher, 93% or higher, 94% or higher, 95% or higher, 96% or higher, 97% or higher, 98% or higher, or 99% or higher, or may be 100%.

The polypeptide according to the present embodiment may be a polypeptide comprising an amino acid sequence a1, a2, a3, a4, or a5 with one or more amino acid residue thereof engineered, and 1 to 20, 1 to 15, 1 to 10, 1 to 7 or 1 to 5 amino acid residues may be engineered. The number of engineered amino acid residues may be 3 or less, may be 2 or less, or may be 1.

The polypeptide according to the present embodiment may be fused with another polypeptide or a protein. Specifically, the polypeptide according to the present embodiment may comprise an amino acid sequence (additional amino acid sequence) other than the sequence having 90% or higher sequence identity to an amino acid sequence represented by SEQ ID NO: 1 with one amino acid residue thereof engineered at any one or both of the N terminus and the C terminus. The additional amino acid sequence may be, for example, a tag sequence.

Examples of a polypeptide or a protein having the additional amino acid sequence include His tag (6 × His, 10 × His, etc.) which is a tag consisting of several (e.g., 6 and 10) His (histidine) residues, streptavidin-bound peptide tag (SBP tag) comprising an amino acid sequence having the ability to bind to a biotin binding site of streptavidin, GST (glutathione S-transferase), HA (influenza hemagglutinin), immunoglobulin constant regions, B-galactosidase, MBP (maltose binding protein), FLAG (Hopp, T.P. et al., BioTechnology (1988) 6, 1204-1210), influenza hemagglutinin (HA), human c-myc fragments, VSV-GP fragments, p18 HIV fragments, T7-tag, HSV-tag, E-tag, SV40T antigen fragments, Ick tag, α-tubulin fragments, B-tag, protein C fragments, Stag, StrepTag, and HaloTag. The His tag may be, for example, the amino acid sequence represented by SEQ ID NO: 11. The SBP tag may be, for example, the amino acid sequence represented by SEQ ID NO: 12.

The polypeptide according to the present embodiment may comprise one or more selected from the group consisting of a streptavidin-bound peptide tag sequence and a His tag sequence at any one or both of the N terminus and the C terminus, and may comprise a streptavidin-bound peptide tag sequence (SBP tag) and a His tag sequence at the C terminus.

The polypeptide according to the present embodiment may comprise amino acid sequence a1 represented by SEQ ID NO: 4 wherein the amino acid residue represented by X is a valine residue (mutation: M141V), amino acid sequence a2 represented by SEQ ID NO: 4 wherein the amino acid residue represented by X is a tyrosine residue (mutation: M141Y), amino acid sequence a3 represented by SEQ ID NO: 6 wherein the amino acid residue represented by

X is a leucine residue (mutation: H182L), amino acid sequence a4 represented by SEQ ID NO: 8 wherein the amino acid residue represented by X is a histidine residue (mutation: W253H), or amino acid sequence a5 represented by SEQ ID NO: 9 wherein the amino acid residue represented by X is a glutamic acid residue (mutation: K260E), and a tag sequence. The polypeptide according to the present embodiment may comprise, in addition to the amino acid sequence a1, a2, a3, a4, or a5 and a tag sequence, a linker sequence that links the amino acid sequence a1, a2, a3, a4, or a5 to the tag sequence. The linker sequence may comprise, for example, the amino acid sequence represented by GGSS or GGS.

The polypeptide according to one embodiment may be a polypeptide X1 constituted by the amino acid sequence a1, a2, a3, a4, or a5, a linker sequence that binds to the C terminus of the amino acid sequence a1, a2, a3, a4, or a5 and is represented by the amino acid sequence: GGSS, and a His tag that binds to the linker sequence and represented by SEQ ID NO: 11. One or more amino acid residues in the polypeptide X1 may be engineered, and 1 to 20, 1 to 15, 1 to 10, 1 to 7, or 1 to 5 amino acid residues may be engineered. The number of engineered amino acid residues in the polypeptide X1 may be 3 or less, may be 2 or less, or may be 1.

The polypeptide according to one embodiment may be a polypeptide X2 constituted by the amino acid sequence a1, a2, a3, a4, or a5, an SBP tag that binds to the C terminus of the amino acid sequence a1, a2, a3, a4, or a5 and is represented by SEQ ID NO: 12, a His tag represented by SEQ ID NO: 11, and a linker sequence that links the His tag and the SBP tag and is represented by the amino acid sequence: GGS. One or more amino acid residues in the polypeptide X2 may be engineered, and 1 to 20, 1 to 15, 1 to 10, 1 to 7 or 1 to 5 amino acid residues may be engineered. The number of engineered amino acid residues in the polypeptide X2 may be 3 or less, may be 2 or less, or may be 1.

The polypeptide according to the present embodiment may be used as a mixture with another polypeptide and may be used in an isolated or generated state. In the case of using the polypeptide as a mixture with another polypeptide, the amino acid of interest may be obtained through the step of isolating or purifying the target product.

The number of amino acid residues in the polypeptide according to the present embodiment in the form of a monomer may be 300 or more, 310 or more, 320 or more, or 325 or more, or may be 330. The number may be 400 or less, 390 or less, 380 or less, or 375 or less. The number of amino acid residues in the polypeptide may be 300 or more and 400 or less.

When the additional amino acid sequence is added to neither the N terminus nor the C terminus, the number of amino acid residues in the polypeptide according to the present embodiment may be 300 or more, 310 or more, 320 or more, or 325 or more, or may be 330. The number may be 360 or less, 350 or less, 340 or less, or 335 or less.

When the additional amino acid sequence is added to any one or both of the N terminus and the C terminus, the number of amino acid residues in the polypeptide according to the present embodiment may be 340 or more, 350 or more, 360 or more, or 370 or more, or may be 374. The number may be 400 or less, 390 or less, 380 or less, or 375 or less.

The polypeptide according to the present embodiment may be used as a monomer or may be used in an associated form of two or more monomers. The polypeptide according to the present embodiment may be a homodimer.

When the polypeptide according to the present embodiment is a homodimer, the homodimer has two times the number of amino acid residues in the form of a monomer.

The peptide compound having a protective group at the N terminus may be a peptide compound consisting of amino acids of 30 residues or less, 25 residues or less, 20 residues or less, 15 residues or less, 14 residues or less, 13 residues or less, 12 residues or less, 11 residues or less, 10 residues or less, 9 residues or less, 8 residues or less, 7 residues or less, 6 residues or less, 5 residues or less, 4 residues or less, 3 residues or less, or 2 residues or less, and 2 residues or more, 3 residues or more, 4 residues or more, 5 residues or more, 6 residues or more, 7 residues or more, 8 residues or more, 9 residues or more, 10 residues or more, 11 residues or more, 12 residues or more, 13 residues or more, 14 residues or more, or 15 residues or more. The peptide compound having a protective group at the N terminus may also be a peptide compound consisting of amino acids of 2 to 10 residues, 2 to 8 residues, 2 to 6 residues, or 2 to 4 residues. The peptide compound having a protective group at the N terminus may also be a peptide compound consisting of amino acids of 5 to 30 residues, 7 to 25 residues, 8 to 15 residues, 9 to 14 residues, 10 to 13 residues, or 11 residues. Another example thereof is a peptide compound comprising a residue of the amino acid (1) at the N terminus.

In the step (B), a purified product may be precipitated so as to reduce the content of semi-specific impurities contained in the precipitate (purified product) obtained in the step (B). In the step (B), the purified product may be precipitated such that the purity of the purified product is 95% by mol or higher. The purity is more preferably 96% by mol or higher, further preferably 97% by mol or higher, still further preferably 98% by mol or higher, particularly preferably 99% by mol or higher, most preferably 100% by mol. In the present specification, the "purity" means the abundance ratio of the substance of interest contained in a substance whose purity is to be measured in the broad sense, and means the ratio of the amount of substance of the lithium salt of the purification target based on the total amount of substance of the lithium salt of the purification target and lithium salt of specific impurities contained in the purified product in the narrow sense.

In the step (B), the purified product may be precipitated such that the content percentage of impurities in the purified product is 5% by mol or lower. The content percentage of impurities is preferably 4% by mol or lower, more preferably 3% by mol or lower, further preferably 2% by mol or lower, particularly preferably 1% by mol or lower, most preferably 0% by mol. In the present specification, the "content percentage of impurities" means the amount of substance

of lithium salt of specific impurities based on the total amount of substance of the lithium salt of the purification target and the lithium salt of specific impurities contained in the purified product.

The purity or the content percentage of impurities may be measured by use of, for example, a nuclear magnetic resonance apparatus, gas chromatography, or high-performance liquid chromatography. In the case of using high-performance liquid chromatography, the purity or the content percentage of impurities can be measured from the area ratio of a UV absorption peak. The following expression 1a represents an expression to determine the purity, and the following expression 2a represents an expression to determine the content percentage of impurities.

[Expression 1]

$$\text{Purity} = \frac{\text{UV absorption peak area of the lithium salt of the purification target} \times \text{Amount of substance per unit UV absorption peak area of the lithium salt of the purification target}}{\begin{array}{c}(\text{UV absorption peak area of the lithium salt of specific impurities} \times \text{Amount of substance per unit UV absorption peak area of the lithium salt of specific impurities} \\ + \text{UV absorption peak area of the lithium salt of the purification target} \times \text{Amount of substance per unit UV absorption peak area of the lithium salt of the purification target})\end{array}} \times 100\ (\%\ \text{by mol}) \ \ldots \text{Expression 1a}$$

[Expression 2]

$$\text{Content percentage of impurities} = \frac{\text{UV absorption peak area of the lithium salt of specific impurities} \times \text{Amount of substance per unit UV absorption peak area of the lithium salt of specific impurities}}{\begin{array}{c}(\text{UV absorption peak area of the lithium salt of specific impurities} \times \text{Amount of substance per unit UV absorption peak area of the lithium salt of specific impurities} \\ + \text{UV absorption peak area of the lithium salt of the purification target} \times \text{Amount of substance per unit UV absorption peak area of the lithium salt of the purification target})\end{array}} \times 100\ (\%\ \text{by mol}) \ \ldots \text{Expression 2a}$$

In this context, the amount of substance per unit UV absorption peak area is a value obtained by dividing the UV absorption peak area of the purification target or the specific impurities by the amount of substance.

In the step (B), the purified product may be precipitated such that the apparent purity of the purified product is 95% or higher. The apparent purity is more preferably 96% or higher, further preferably 97% or higher, still further preferably 98% or higher, particularly preferably 99% or higher, most preferably 100%. In the present specification, the "apparent purity" means the ratio of the UV absorption peak area at a particular wavelength of the purification target based on the total UV absorption peak area at the particular wavelength of the purification target and specific impurities measured after separation of the purified product into the purification target and the specific impurities by high-performance liquid chromatography.

In the step (B), the purified product may be precipitated such that the apparent content percentage of impurities of the purified product is 5% or lower. The apparent content percentage of impurities is preferably 4% or lower, more preferably 3% or lower, further preferably 2% or lower, particularly preferably 1% or lower, most preferably 0%. In the present specification, the "apparent content percentage of impurities" means the ratio of the UV absorption peak area at a particular wavelength of specific impurities based on the total UV absorption peak area at the particular wavelength of the purification target and the specific impurities measured after separation of the purified product into the purification target and the specific impurities by high-performance liquid chromatography.

The following expression 1b represents an expression to determine the apparent purity, and the following expression 2b represents an expression to determine the apparent content percentage of impurities.

[Expression 3]

$$\text{Apparent purity} = \frac{\text{UV absorption peak area at the particular wavelength of the lithium salt of the purification target}}{\begin{array}{c}(\text{UV absorption peak area at the particular wavelength of the lithium salt of the purification target} \\ + \text{UV absorption peak area at the particular wavelength of the lithium salt of specific impurities})\end{array}} \times 100\ (\%) \ldots \text{Expression 1b}$$

[Expression 4]

$$\text{Apparent content percentage of impurities} = \frac{\text{UV absorption peak area at the particular wavelength of the lithium salt of specific impurities}}{\begin{array}{c}(\text{UV absorption peak area at the particular wavelength of the lithium salt of the purification target} \\ + \text{UV absorption peak area at the particular wavelength of the lithium salt of specific impurities})\end{array}} \times 100\ (\%) \ldots \text{Expression 2b}$$

In the present specification, the particular wavelength is a wavelength within ±10% of the maximum absorption

wavelength exhibited by the protective group of the purification target. When a plurality of maximum absorption wavelengths are present, any maximum absorption wavelength that exhibits 30% or more absorption intensity based on absorption intensity at the largest absorption wavelength among them may be selected as the maximum absorption wavelength. The influence of noise can be reduced by selecting a longer wavelength from among a plurality of maximum absorption wavelengths. A sample concentration can be adjusted depending on absorption intensity at the selected maximum absorption wavelength. For measurement, a maximum absorption wavelength that seems most preferable from the viewpoint of easy adjustment of a sample concentration or noise reduction can be selected.

For example, UV light at 254 nm for a Fmoc group, 197 nm for a Boc group, or 210 nm for a Cbz group can be used as UV light at the particular wavelength.

In the production method according to one embodiment, the content percentage of β-alanine or a derivative thereof in the mixture to be purified can be reduced by obtaining lithium salt. In other words, the production method according to the present embodiment may comprise the step of reducing the content percentage of β-alanine or a derivative thereof.

The production method according to the present embodiment may further comprise the step of preparing a salt other than the lithium salt. Specifically, the lithium salt obtained by the production method according to one embodiment may be desalted, for example, to obtain an amino acid in a free form or a peptide compound in a free form. The desalting can be performed by a known method. Further, a salt other than the lithium salt can be produced from the obtained amino acid or peptide compound in a free form. Examples of the salt other than the lithium salt can include: salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals (sodium or potassium); salts with alkaline earth metals such as calcium and magnesium; and ammonium salt.

The production method according to one embodiment can produce a peptide compound having a number of amino acid residues of interest, for example a peptide compound having a cyclic moiety (cyclic peptide compound), by linking the obtained amino acid or peptide compound in a free form with an amino acid or peptide compound in accordance with a routine method.

The number of amino acid residues in the cyclic peptide compound according to the present embodiment may be 30 or less, 25 or less, 20 or less, 15 or less, 14 or less, 13 or less, 12 or less, 11 or less, 10 or less, 9 or less, 8 or less, 7 or less, 6 or less, 5 or less, 4 or less, 3 or less, or 2 or less. The cyclic peptide compound according to the present embodiment may be a peptide compound consisting of 2 or more, 3 or more, 4 or more, 5 or more, 6 or more, 7 or more, 8 or more, 9 or more, 10 or more, 11 or more, 12 or more, 13 or more, 14 or more, or 15 or more amino acids. The number of amino acid residues in the cyclic peptide compound according to the present embodiment may be between 2 and 10, between 2 and 8, between 2 and 6, or between 2 and 4. The number of amino acid residues in the cyclic peptide compound according to the present embodiment may be 5 or more and 30 or less, 7 or more and 25 or less, 8 or more and 15 or less, 9 or more and 14 or less, 10 or more and 13 or less, or 11.

The "cyclic moiety" of the cyclic peptide compound means a cyclic portion formed by the linkage of two or more amino acid residues. The number of amino acid residues constituting the cyclic moiety of the cyclic peptide compound may be between 5 and 14, between 6 and 14, between 7 and 14, between 8 and 14, between 9 and 14, between 10 and 14, between 5 and 13, between 6 and 13, between 7 and 13, between 8 and 13, between 9 and 13, between 10 and 13, between 5 and 12, between 6 and 12, between 7 and 12, between 8 and 12, between 9 and 12, between 10 and 12, between 5 and 11, between 6 and 11, between 7 and 11, between 8 and 11, between 9 and 11, between 10 and 11, or 11. The cyclic moiety is preferably formed via a covalent bond such as an amide bond, carbon-carbon bond formation reaction, a S-S bond, a thioether bond, or a triazole bond, and may be formed by bonding the N-terminal group of a linear peptide compound having linked amino acid residues to the C-terminal group thereof. Specifically, the cyclic moiety can be formed, for example, by bonding the N-terminal amino group of a linear peptide compound having linked amino acid residues to the C-terminal carboxyl group thereof. The cyclization may be in any form such as cyclization through a carbon-nitrogen bond (e.g., an amide bond), cyclization through a carbon-oxygen bond (e.g., an ester bond and an ether bond), cyclization through a carbon-sulfur bond (e.g., a thioether bond), cyclization through a carbon-carbon bond, cyclization through a sulfur-sulfur bond, or cyclization by heterocyclic ring construction. Among them, cyclization via a covalent bond such as an amide bond or a carbon-carbon bond is preferred, and cyclization via the amide bond between a side chain carboxy group and a backbone amino group is more preferred. The carboxy group and the amino group, etc. for use in cyclization may be positioned on the backbone or may be positioned on a side chain without particular limitations as long as the positions permit cyclization.

The number of ring atoms in the cyclic peptide compound according to the present embodiment may be between 15 and 46. In the present specification, the "number of ring atoms" means the number of atoms in a cyclic compound (ring atoms) including the innermost portion of the ring. When the compound has a plurality of rings, the number of ring atoms is defined as the number of ring atoms in a ring having the largest number of ring atoms. When two rings share some atoms, a ring having a smaller number of shared atoms is used to calculate the number of ring atoms in each ring. The "number of

ring atoms" will be further described with reference to specific examples. According to this method used, for example, the number of ring atoms in tetrahydrofuran (THF) is 5, and the number of ring atoms in tacrolimus (FK506) is 21.

The number of ring atoms in the cyclic peptide compound according to the present embodiment may be, for example, 34 or more and 46 or less, 34 or more and 43 or less, 34 or more and 40 or less, 34 or more and 37 or less, 34 or more and 36 or less, or 34. The ring atoms for use in the calculation of the number of ring atoms may be selected from the group consisting of a carbon atom, a hydrogen atom, a nitrogen atom, an oxygen atom, a sulfur atom, a phosphorus atom, and a silicon atom or may be selected from the group consisting of a carbon atom, a hydrogen atom, a nitrogen atom, and an oxygen atom.

The cyclic peptide compound according to the present embodiment may have a linear moiety in addition to the cyclic moiety. A specific aspect of the number of amino acid residues in the cyclic peptide compound is the same as that of the number of amino acid residues in the peptide compound mentioned above. When the cyclic peptide compound has a linear moiety, the total number of amino acid residues in the cyclic moiety and the linear moiety preferably falls within the presented range. When the cyclic peptide compound has a linear moiety, the number of amino acid residues constituting the cyclic moiety is preferably between 5 and 15, between 6 and 15, between 6 and 14, between 7 and 14, between 8 and 14, or between 7 and 13, more preferably between 7 and 12 or between 8 and 11, further preferably between 9 and 11, particularly preferably between 10 or 11, and the number of amino acid residues constituting the linear moiety is preferably between 1 and 8, between 1 and 7, between 1 and 6, between 1 and 5, or between 1 and 4, more preferably between 1 and 3.

The molecular weight of the cyclic peptide compound according to the present embodiment is not particularly limited and may be, for example, 500 or higher, 550 or higher, 600 or higher, 650 or higher, 700 or higher, 750 or higher, 800 or higher, 850 or higher, 900 or higher, or 950 or higher and is preferably 1000 or higher, 1100 or higher, 1200 or higher, 1300 or higher, or 1400 or higher. The upper limit of the molecular weight of the peptide compound according to the present embodiment is not particularly limited and is preferably 5000 or lower, 4000 or lower, 3000 or lower, 2500 or lower, or 2000 or lower. In the present specification, the molecular weight means the total sum of atomic weights of atoms constituting a compound molecule (unit: "g/mol") and is obtained by calculating the total sum of atomic weights of atoms included in a molecular structural formula (unit: "g/mol"). In the present specification, the unit of the molecular weight may be omitted. The molecular weight of the peptide compound can be calculated by liquid chromatography mass spectrometry (LC/MS) described in Examples. The specific aspects of the peptide compound described above can be applied without limitation to (i) the peptide compound containing one or more N-substituted amino acid residues according to the present embodiment, as long as the peptide compound is a peptide compound containing one or more N-substituted amino acid residues.

The cyclic peptide compound according to the present embodiment may contain one or more N-substituted amino acid residues, preferably contains at least three N-substituted amino acid residues, more preferably contains at least four N-substituted amino acid residues, and further preferably contains at least five N-substituted amino acid residues. The N-substituted amino acid residues may be present continuously or discontinuously in the N-substituted cyclic peptide compound.

[Examples]

The present invention will be further illustrated with reference to Examples given below and however is not limited by these examples. The starting materials for amino acid derivatives represented by tetrahydrofuran, acetonitrile, aqueous lithium hydroxide solution, lithium tert-butoxide tetrahydrofuran solution, and (S)-2-(tert-butoxycarbonylamino)-3-phenylpropanoic acid used in carrying out the present invention were obtained from commercial suppliers other than those specifically described, and used without purification. In Examples, the abbreviations described in Tables 1 and 2 were used.

[Table 1]

| Abbreviation | Name |
|---|---|
| Boc | tert-Butoxycarbonyl |
| Cbz | Benzyloxycarbonyl |
| Fmoc | 9-Fluorenylmethyloxycarbonyl |
| NADP+ | Oxidized nicotinamide adenine dinucleotide phosphate |
| NMAADH | N-methyl-amino acid dehydrogenase |

[Table 2]

| Abbreviation | Name | Structural formula |
|---|---|---|
| Boc-Phe-OH | (S)-2-(tert-Butoxycarbonylamino)-3-phenylpropanoic acid | 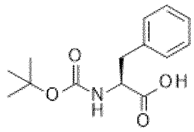 |
| Boc-β-Ala-OH | 3-(tert-Butoxycarbonylamino)propanoic acid | 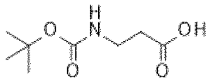 |
| Cbz-β-Ala-OH | 3-(Benzyloxycarbonylamino)propanoic acid | 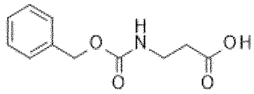 |
| Cbz-MeGly(cPent)-OH | (S)-2-(Benzyloxycarbonyl(methyl)amino)-2-cyclopentylacetic acid | 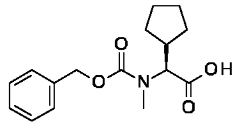 |
| Cbz-MePhe-OH | (S)-2-(Benzyloxycarbonyl(methyl)amino)-3-phenylpropanoic acid | 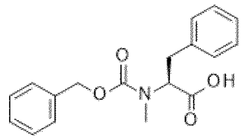 |
| Fmoc-β-Ala-OH | 3-(9H-Fluoren-9-ylmethoxycarbonylamino)propanoic acid | 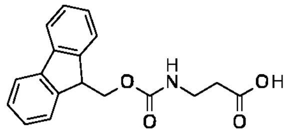 |
| Fmoc-β-Phg-OH | (R)-3-(9H-Fluoren-9-ylmethoxycarbonylamino)-3-phenylpropanoic acid | 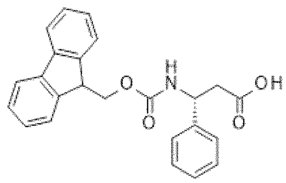 |
| Fmoc-Pro-OH | (S)-1-(9H-Fluoren-9-ylmethoxycarbonyl)pyrrolidine-2-carboxylic acid | 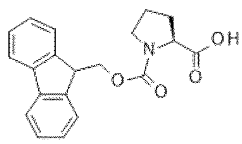 |

Condition 1 of high-performance liquid chromatography

Apparatus: Waters AQUITY H-class/QDa
Column: (I.D. x length (mm), particle size (μm)): ACQUITY UPLC HSS T3 (2.1 x 50, 1.8)
Mobile phase: A) 0.05% TFA H2O, B) 0.05% TFA MeCN
Gradient (A/B): 100/0 to 2/98 (5.0 min) → 2/98 (1.0 min) → 100/0 (0.01 min) → 100/0 (2.0 min)
Flow rate (ml/min): 0.5
Column temperature: 30°C

Condition 2 of high-performance liquid chromatography

Apparatus: Waters AQUITY H-class
Column: (I.D. x length (mm), particle size (μm)): Ascentis Express RP-Amide (2.1 x 50, 2.7)
Mobile phase: A) 0.05% TFA H2O, B) 0.05% TFA MeCN
Gradient (A/B): 95/5 to 0/100 (4.0 min) → 0/100 (0.5 min) → 95/5 (0.1 min) → 95/5 (1.4 min)

Flow rate (ml/min): 0.5
Column temperature: 35°C

Condition 3 of high-performance liquid chromatography

Apparatus: Waters AQUITY H-class/QDa
Column: (I.D. x length (mm), particle size (μm)): Ascentis Express C18 (3.0 x 50, 2.7)
Mobile phase: A) 0.05% TFA H2O, B) 0.05% TFA MeCN
Gradient (A/B): 95/5 to 0/100 (5.0 min) → 0/100 (1.0 min) → 95/5 (0.01 min) → 95/5 (2.0 min)
Flow rate (ml/min): 0.5
Column temperature: 30°C

Condition 4 for high-performance liquid chromatography

Apparatus: Waters AQUITY H-class / QDa
Column (I.D. x length (mm), particle size (μm)): DAICEL CHIRALPAK IC-3 (4.6 x 150, 3)
Mobile phase : A) 0.05% TFA H2O, B) 0.05% TFA MeCN
Gradient (A/B): 95/5 to 40 / 60 (20.0 min) → 95/5 (0.1 min) → 95/5 (4.9 min)
Flow Rate (ml / min): 1.0
Column temperature : 30°C

Preparation Example 1: Preparation of W253H-SBP-His

A gene of amino acid sequence represented by SEQ ID NO: 8, in which X is His, C-terminally tagged with a streptavidin-binding peptide tag sequence (GTDEKTTGWRGGHVVEGLAGELEQLRARLEHHPQ), a linker sequence (GGS), and a His tag sequence (HHHHHH) was synthesized and cloned into a vector for expression in E. coli. This expression vector was transferred to a BL21 (DE3) E. coli strain (Novagen), which was then cultured at 18°C for 2 days using Overnight Express Instant TB Medium (Novagen) to express the protein of interest, the final preparation (SEQ ID NO: 10, W253H).

Synthesis Example 1: Synthesis of partially purified (S)-2-(benzyloxycarbonyl(methyl)amino)-2-cyclopentyl-acetic acid

[Formula 2]

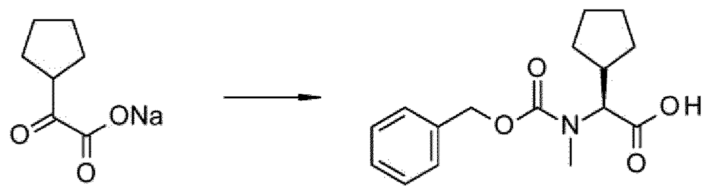

2-Cyclopentyl-2-oxo-acetic acid sodium salt (3.9 g, 24 mmol), methylamine hydrochloride (8.1 g, 120 mmol), D-glucose (8.7 g, 48 mmol), and N,N-di(2-hydroxyethyl)glycine (7.8 g, 48 mmol) were dissolved in distilled water (50 mL), and the outside temperature of the reaction container was set to 25°C. The pH of the solution was adjusted to 8.8 by the addition of a 5 M aqueous sodium hydroxide solution (6.0 mL). NADP+ (0.18 g, 0.24 mmol), D-glucose dehydrogenase (3.8 mg, 960 U, 250 U/mg), and an NMAADH variant enzyme solution (final preparation, 1.6 mL; corresponding to 43 mg in terms of the enzyme, 1.1% by mass) were added thereto, and the mixture was stirred at an outside temperature of 25°C of the reaction container for 24 hours. The pH of the solution was adjusted to 1.9 by the addition of concentrated hydrochloric acid (3.0 mL), and the mixture was stirred for 1 hour. Insoluble matter was filtered off through a celite pad, and the celite pad was washed with distilled water (20 mL) to give a (S)-2-cyclopentyl-2-(methylamino)acetic acid solution (120 g).

Mass spectrometry (MS (ESI)) was performed under the condition 1 of high-performance liquid chromatography. A peak with m/z = 158.1 $(M + H)^+$ was confirmed at a retention time of 1.5 minutes to confirm that the (S)-2-cyclopentyl-2-(methylamino)acetic acid solution of interest was obtained.

The pH of the (S)-2-cyclopentyl-2-(methylamino)acetic acid solution (60 g; corresponding to 12 mmol) was adjusted to 12.0 by the addition of a 10 M aqueous sodium hydroxide solution (10 mL). The obtained solution was

concentrated into dryness under reduced pressure at an outside temperature of 40°C of the reaction container. Distilled water (30 mL) was added thereto, and the pH of the solution was adjusted to pH 9.7 by the addition of concentrated hydrochloric acid (0.75 mL). Acetonitrile (10 mL), N-carbobenzoxyoxysuccinimide (4.2 g, 17 mmol), and methyl tert-butyl ether (10 mL) were added thereto, and the mixture was stirred for 1 hour. A 50 w/v% aqueous sodium hydroxide solution (1.5 mL) was added thereto, and the mixture was stirred for 3 hours. N-Carbobenzoxyoxysuccinimide (1.2 g, 4.8 mmol) was added thereto, and the mixture was stirred for 90 minutes. A 50 w/v% aqueous sodium hydroxide solution (0.5 mL) was added thereto, and the mixture was stirred for 18 hours. The title compound was extracted into an organic layer by the addition of concentrated hydrochloric acid (8.0 mL). The obtained organic layer was concentrated into dryness under reduced pressure at an outside temperature of 40°C of the reaction container. Methyl tert-butyl ether (15 mL) was added thereto, and the organic layer was washed twice with a 5% aqueous disodium hydrogen phosphate solution (10 mL × 2) and once with a 2 M aqueous sodium hydroxide solution (10 mL) to extract the title compound into an aqueous layer. The obtained aqueous layers were combined, and the title compound was reextracted into an organic layer by the addition of methyl tert-butyl ether (20 mL) and concentrated hydrochloric acid (1.5 mL). The obtained organic layer was concentrated into dryness under reduced pressure at an outside temperature of 40°C of the reaction container. The title compound was extracted into an aqueous layer by the addition of methyl tert-butyl ether (30 mL) and a 1 M aqueous sodium hydroxide solution (30 mL). The obtained aqueous layer was washed with methyl tert-butyl ether (30 mL). The title compound was reextracted into an organic layer by the addition of toluene (30 mL) and concentrated hydrochloric acid (3.0 mL). The obtained organic layer was concentrated into dryness under reduced pressure at an outside temperature of 40°C of the reaction container to give partially purified (S)-2-(benzyloxycarbonyl(methyl)amino)-2-cyclopentyl-acetic acid (2.8 g).

Synthesis Example 2: Synthesis of partially purified (S)-2-(benzyloxycarbonyl(methyl)amino)-2-cyclopentyl-acetic acid

[Formula 3]

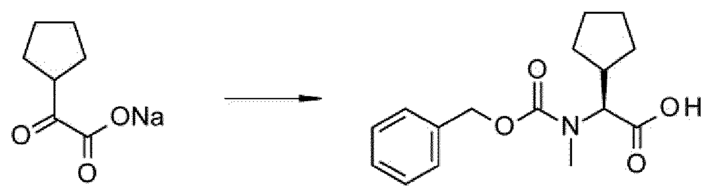

2-Cyclopentyl-2-oxo-acetic acid sodium salt (5.3 g, 33 mmol), methylamine hydrochloride (11 g, 160 mmol), D-glucose (12 g, 65 mmol), and N,N-di(2-hydroxyethyl)glycine (11 g, 65 mmol) were dissolved in distilled water (69 mL), and the outside temperature of the reaction container was set to 25°C. The pH of the solution was adjusted to 9.2 by the addition of a 50 w/v% aqueous sodium hydroxide solution (4.8 mL). NADP+ (0.25 g, 0.33 mmol), D-glucose dehydrogenase (26 mg, 1300 U, 50 U/mg), and the NMAADH variant enzyme solution (final preparation, 2.0 mL; corresponding to 53 mg in terms of the enzyme, 1.0% by mass) were added thereto, and the mixture was stirred at an outside temperature of 25°C of the reaction container for 21 hours (rate of reaction conversion: 99% or more). The pH of the solution was adjusted to 1.9 by the addition of concentrated hydrochloric acid (7.5 mL), and the mixture was stirred for 1 hour. Insoluble matter was filtered off through a celite pad, and the celite pad was washed with distilled water (11 mL) to give a (S)-2-cyclopentyl-2-(methylamino)acetic acid solution (130 g).

Mass spectrometry (MS (ESI)) was performed under the condition 1 of high-performance liquid chromatography. A peak with m/z = 158.1 $(M + H)^+$ was confirmed at a retention time of 1.4 minutes to confirm that the (S)-2-cyclopentyl-2-(methylamino)acetic acid solution of interest was obtained.

The pH of the (S)-2-cyclopentyl-2-(methylamino)acetic acid solution (27 g; corresponding to 6.5 mmol) was adjusted to 12.3 by the addition of a 50 w/v% aqueous sodium hydroxide solution (3.2 mL). The obtained solution was concentrated into dryness at an outside temperature of 40°C of the reaction container. The pH of the solution was adjusted to pH 9.2 by the addition of distilled water (24 mL) and concentrated hydrochloric acid (0.53 mL) at an outside temperature of 25°C of the reaction container. Methyl tert-butyl ether (5.3 mL), acetonitrile (2.7 mL), and N-carbobenzoxyoxysuccinimide (1.8 g, 7.2 mmol) were added thereto, and the mixture was stirred for 105 minutes. Acetonitrile (1.6 mL) was added thereto, and the mixture was further stirred for 30 minutes. The pH of the solution was adjusted to 9.3 by the addition of a 50 w/v% aqueous sodium hydroxide solution (0.8 mL), and the mixture was stirred for 105 minutes. N-Carbobenzoxyoxysuccinimide (1.0 g, 3.9 mmol) was added thereto, and the mixture was stirred for 105 minutes. The pH of the solution was adjusted to 9.2 by the addition of a 50 w/v% aqueous sodium hydroxide solution (0.5 mL). Then, N-carbobenzoxyoxysuccinimide (1.1 g, 4.6 mmol) was added thereto, and the mixture was stirred for 13 hours. The pH of the solution was adjusted to 0.4 by the addition of concentrated hydrochloric acid (4.3 mL). Then, the title compound was extracted into an

organic layer by the addition of methyl tert-butyl ether (3.0 mL). The title compound was extracted into an organic layer by the addition of methyl tert-butyl ether (5.3 mL) to the obtained aqueous layer. The obtained organic layers were combined and rendered basic by the addition of a 2 M aqueous sodium hydroxide solution (11 mL). The title compound was extracted into an aqueous layer by the addition of methyl tert-butyl ether (5.3 mL). Methyl tert-butyl ether (11 mL) was added to the aqueous layer for washing. The pH of the aqueous layer was adjusted to 0.0 by the addition of methyl tert-butyl ether (11 mL) and concentrated hydrochloric acid (2.1 mL). The title compound was extracted into an organic layer to give a partially purified (S)-2-(benzyloxycarbonyl(methyl)amino)-2-cyclopentyl-acetic acid solution (14 g).

Mass spectrometry (MS (ESI)) was performed under the condition 1 of high-performance liquid chromatography. A peak with m/z = 292.1 $(M + H)^+$ was confirmed at a retention time of 3.9 minutes to confirm that the partially purified (S)-2-(benzyloxycarbonyl(methyl)amino)-2-cyclopentyl-acetic acid of interest was obtained.

Synthesis Example 3: Method for synthesizing (S)-2-(benzyloxycarbonyl(methyl)amino)-2-cyclopentyl-acetic acid lithium salt seed crystals

(S)-2-(Benzyloxycarbonyl(methyl)amino)-2-cyclopentyl-acetic acid (1.0 g, 3.4 mmol, commercially available product) was dissolved in tetrahydrofuran (5.0 ml, 5.0 v/w), and the solution was stirred at an outside temperature of 25°C of the reaction container. Lithium tert-butoxide (0.30 g, 3.8 mmol) was added thereto, and precipitated crystals were confirmed. Tetrahydrofuran (20 ml, 20 v/w) was added thereto, and the mixture was stirred for 1 hour. The crystals were filtered, and the obtained crystals were washed with tetrahydrofuran (5.0 ml, 5.0 v/w). The crystals were dried under reduced pressure at an outside temperature of 40°C of the reaction container for 3 hours to give (S)-2-(benzyloxycarbonyl(methyl)amino)-2-cyclopentyl-acetic acid lithium salt (0.90 g, 88% yield).

Comparative Example 1 and Example 1: Purification of amino acid derivative in free form and in lithium salt form

The purification of an amino acid derivative was compared between a free form (carboxylic acid) and lithium salt. An amino acid derivative containing impurities was isolated in a free form or in a lithium salt form, and apparent purity was compared therebetween. The apparent purity or the apparent content percentage of impurities was calculated from a UV absorption peak area ratio of high-performance liquid chromatography. The apparent purity was calculated according to the expression 1b, and the apparent content percentage of impurities was calculated according to the expression 2b.

Comparative Example 1: Purification of (S)-2-(benzyloxycarbonyl(methyl)amino)-2-cyclopentyl-acetic acid (free form)

The partially purified (S)-2-(benzyloxycarbonyl(methyl)amino)-2-cyclopentyl-acetic acid (1.4 g, theoretical yield quantity as (S)-2-(benzyloxycarbonyl(methyl)amino)-2-cyclopentyl-acetic acid: 1.8 g; corresponding to 6.0 mmol) obtained in Synthesis Example 1 was dissolved in toluene (3.0 mL, 1.7 v/w), and the solution was stirred at an outside temperature of 25°C of the reaction container using a stirring blade. Heptane (1.0 mL, 0.6 v/w) and seed crystals (crystals of (S)-2-(benzyloxycarbonyl(methyl)amino)-2-cyclopentyl-acetic acid (free form), commercially available product, 5.0 mg) were added thereto. The mixture was stirred for 15 minutes, and precipitated crystals were confirmed. Heptane (5.5 mL, 3.1 v/w) was added dropwise thereto over 1 hour, and the mixture was further stirred for 30 minutes. The mixture was further stirred at an outside temperature of 0°C of the reaction container for 1 hour. The crystals were filtered, and the obtained crystals were washed with heptane/toluene (v/v) = 4/1 (4.0 mL, 2.3 v/w). The crystals were dried under reduced pressure at an outside temperature of 40°C of the reaction container for 1 hour to give (S)-2-(benzyloxycarbonyl(methyl)amino)-2-cyclopentyl-acetic acid (1.3 g, 73% yield (calculated based on the 2-cyclopentyl-2-oxo-acetic acid sodium salt of Synthesis Example 1)).

The structure was confirmed by mass spectrometry (MS (ESI)) under the condition 3 of high-performance liquid chromatography. The peak with m/z = 224.1 $(M + H)^+$ of Cbz-β-Ala-OH, one of the impurities, was confirmed at a retention time of 2.6 minutes. The peak with m/z = 292.1 $(M + H)^+$ of Cbz-MeGly(cPent)-OH was confirmed at a retention time of 3.9 minutes. The UV absorption peak areas of Cbz-MeGly(cPent)-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content percentage of impurities was performed both for the partially purified (S)-2-(benzyloxycarbonyl(methyl)amino)-2-cyclopentyl-acetic acid obtained in Synthesis Example 1 (before purification) and for the (S)-2-(benzyloxycarbonyl(methyl)amino)-2-cyclopentyl-acetic acid obtained in Comparative Example 1 (after purification). The results are shown in Table 3.

[Table 3]

| | Cbz-β-Ala-OH (Apparent content percentage of impurities) | Cbz-MeGly(cPent)-OH (Apparent purity) |
|---|---|---|
| Before purification | 1.5% | 98.5% |
| After purification | 1.3% | 98.7% |

Example 1-1: Purification of (S)-2-(benzyloxycarbonyl(methyl)amino)-2-cyclopentyl-acetic acid in lithium salt form

[Formula 4]

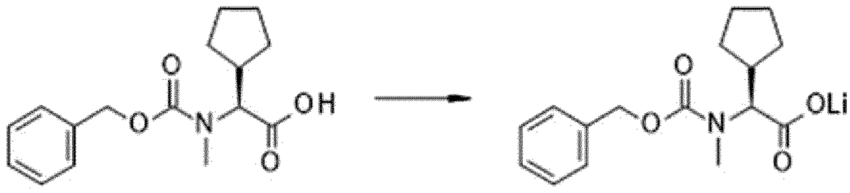

The (S)-2-(benzyloxycarbonyl(methyl)amino)-2-cyclopentyl-acetic acid (500 mg, 1.7 mmol) obtained in Comparative Example 1 was dissolved in acetonitrile (2.5 ml, 5.0 v/w), and the solution was stirred at an outside temperature of 25°C of the reaction container using a stirring blade. A 4 M aqueous lithium hydroxide solution (0.47 ml, 1.9 mmol) and distilled water (0.50 ml, 1.0 v/w) were added thereto, and the mixture was stirred at an outside temperature of 40°C of the reaction container for 20 minutes. Acetonitrile (5.0 ml, 10 v/w) was added thereto over 10 minutes, and seed crystals (synthesized in Synthesis Example 3, 5.0 mg) were added thereto. The mixture was stirred for 40 minutes, and precipitated crystals were confirmed. The outside temperature of the reaction container was set to 25°C, and the mixture was stirred for 3 hours. The outside temperature of the reaction container was set to 0°C, and the mixture was stirred for 30 minutes. Acetonitrile (2.5 ml, 5.0 v/w) was added dropwise thereto over 5 minutes, and the mixture was further stirred for 30 minutes. Acetonitrile (5.0 ml, 10 v/w) was added dropwise thereto over 5 minutes, and the mixture was stirred for 30 minutes. The crystals were filtered, and the obtained crystals were washed with distilled water/acetonitrile (v/v) = 5/95 (2.0 mL, 4.0 v/w). The crystals were dried under reduced pressure at an outside temperature of 40°C of the reaction container for 3 hours to give (S)-2-(benzyloxycarbonyl(methyl)amino)-2-cyclopentyl-acetic acid lithium salt (360 mg, 71% yield, calculated based on the (S)-2-(benzyloxycarbonyl(methyl)amino)-2-cyclopentyl-acetic acid obtained in Comparative Example 1).

Mass spectrometry (MS (ESI)) and apparent purity analysis based thereon were performed under the condition 3 of high-performance liquid chromatography. The peak of Cbz-β-Ala-OH, one of the impurities, was confirmed at a retention time of 2.6 minutes. The peak with m/z = 292.1 $(M + H)^+$ of Cbz-MeGly(cPent)-OH was confirmed at a retention time of 3.9 minutes. The UV absorption peak areas of Cbz-MeGly(cPent)-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content percentage of impurities was performed both for the (S)-2-(benzyloxycarbonyl(methyl)amino)-2-cyclopentyl-acetic acid obtained in Comparative Example 1 (before purification) and for the (S)-2-(benzyloxycarbonyl(methyl)amino)-2-cyclopentyl-acetic acid lithium salt obtained in Example 1-1 (after purification). The results are shown in Table 4.

[Table 4]

| | Cbz-β-Ala-OH (Apparent content percentage of impurities) | Cbz-MeGly(cPent)-OH (Apparent purity) |
|---|---|---|
| Before purification | 1.3% | 98.7% |
| After purification | N.D. | 100% |
| N.D.: not detected | | |

Example 1-2: Purification of (S)-2-(benzyloxycarbonyl(methyl)amino)-2-cyclopentyl-acetic acid in lithium salt form

[Formula 5]

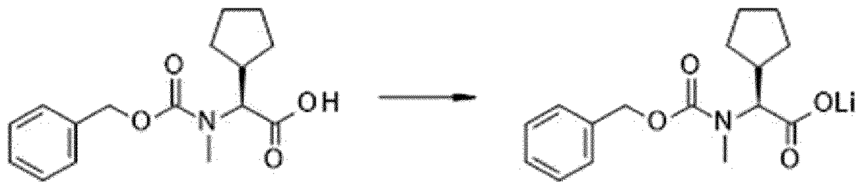

A solution of the crude product (S)-2-(benzyloxycarbonyl(methyl)amino)-2-cyclopentyl-acetic acid (7.0 g, theoretical yield quantity as (S)-2-(benzyloxycarbonyl(methyl)amino)-2-cyclopentyl-acetic acid: 0.95 g; corresponding to 3.3 mmol) obtained in Synthesis Example 2 was concentrated into dryness at an outside temperature of 40°C of the reaction container. Acetonitrile (3.2 mL, 3.4 v/w) was added thereto, and the mixture was stirred at an outside temperature of 25°C of the reaction container using a stirring blade. Distilled water (0.53 mL, 0.6 v/w) and a 4 M aqueous lithium hydroxide solution (0.90 mL, 3.6 mmol) were added thereto, and the mixture was stirred for 20 minutes. Acetonitrile (2.7 mL, 2.8 v/w) was added thereto over 10 minutes, and seed crystals (synthesized in Synthesis Example 3, 5.0 mg) were added thereto. The mixture was stirred for 15 minutes. Acetonitrile (5.3 mL, 5.6 v/w) was added dropwise thereto over 20 minutes, and the mixture was stirred for 17 hours. Acetonitrile (5.3 mL, 5.6 v/w) was added dropwise thereto over 30 minutes, and the mixture was stirred for 1 hour. Acetonitrile (5.3 mL, 5.6 v/w) was added dropwise thereto over 30 minutes, and the mixture was stirred for 1 hour. The crystals were filtered, and the obtained crystals were washed with distilled water/acetonitrile (v/v) = 5/95 (3.2 mL, 3.4 v/w). The crystals were dried under reduced pressure at an outside temperature of 40°C of the reaction container for 2.5 hours to give (S)-2-(benzyloxycarbonyl(methyl)amino)-2-cyclopentyl-acetic acid lithium salt (yield quantity: 620 mg, yield: 64%, calculated based on the 2-cyclopentyl-2-oxo-acetic acid sodium salt of Synthesis Example 2).

Mass spectrometry (MS (ESI)) and apparent purity analysis based thereon were performed under the condition 1 of high-performance liquid chromatography. The peak of Cbz-β-Ala-OH, one of the impurities, was confirmed at a retention time of 2.8 minutes. The peak with m/z = 292.1 $(M + H)^+$ of Cbz-MeGly(cPent)-OH was confirmed at a retention time of 3.9 minutes. The UV absorption peak areas of Cbz-MeGly(cPent)-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content percentage of impurities was performed both for the partially purified (S)-2-(benzyloxycarbonyl(methyl)amino)-2-cyclopentyl-acetic acid solution obtained in Synthesis Example 2 (before purification) and for the (S)-2-(benzyloxycarbonyl(methyl)amino)-2-cyclopentyl-acetic acid lithium salt obtained in Example 1-2 (after purification). The results are shown in Table 5.

[Table 5]

| | Cbz-β-Ala-OH (Apparent content percentage of impurities) | Cbz-MeGly(cPent)-OH (Apparent purity) |
|---|---|---|
| Before purification | 3.6% | 96.4% |
| After purification | N.D. | 100% |
| N.D.: not detected | | |

Chiral analysis was carried out using the condition 4 for high performance liquid chromatography. The measurement wavelength was 210 nm. The retention times of (S) compound and (R) compound were confirmed to be 16.7 min and 17.3 min, respectively, by analysis of a standard sample (purchased from Amatek). As a result of the chiral analysis, the (R) compound was not detected in the (S)-2-(benzyloxycarbonyl (methyl) amino)-2-cyclopentyl-acetic acid lithium salt (after purification) obtained in Example 1-2 and had an optical purity of 99.9% ee or more.

Example 2: Purification of amino acid derivative by lithium salt formation

The purification effect of amino acid derivatives by lithium salt formation was evaluated. To an amino acid derivative having a specific protective group, 3-aminopropanoic acid (β-alanine) having the same protective group was added as impurities to prepare a mixture. The mixture was subjected to isolation and purification by lithium salt formation, and the purification effect was verified by comparing the apparent purities of the mixture and the isolated compound. The apparent content percentage of impurities was calculated using the same method described in Example 1.

Example 2-1: Synthesis of (R)-3-(9H-fluoren-9-ylmethoxycarbonylamino)-3-phenylpropanoic acid lithium salt

[Formula 6]

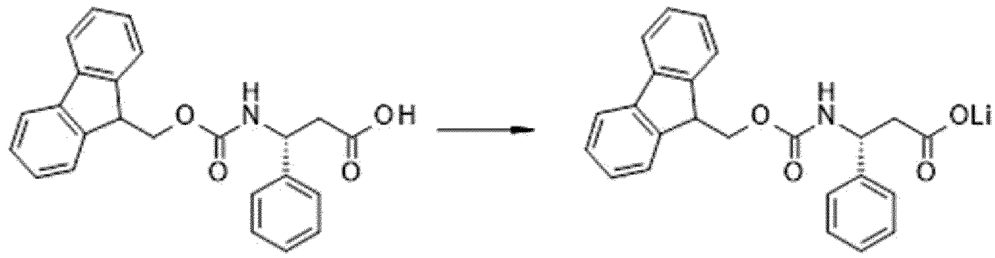

To (R)-3-(9H-fluoren-9-ylmethoxycarbonylamino)-3-phenyl-propanoic acid (500 mg, 1.3 mmol) and 3-(9H-fluoren-9-ylmethoxycarbonylamino)propanoic acid (20 mg, 0.065 mmol), tetrahydrofuran (18 ml, 36 v/w) and distilled water (2.0 ml, 4.0 v/w) were added, and the mixture was stirred at an outside temperature of 25°C of the reaction container using a stirring blade. A 4 M aqueous lithium hydroxide solution (0.081 mL, 0.32 mmol) was added thereto over 3 minutes, and the mixture was stirred for 10 minutes. A 4 M aqueous lithium hydroxide solution (0.081 ml, 0.32 mmol) was added thereto over 3 minutes, and the mixture was stirred for 1 hour, then precipitated crystals were confirmed. Furthermore, a 4 M aqueous lithium hydroxide solution (0.081 ml, 0.32 mmol) was added thereto over 3 minutes, and a stirring operation for 1 hour was repeated twice. The crystals were filtered, and the obtained crystals were washed with distilled water/-tetrahydrofuran (v/v) = 10/90 (5.0 mL, 10 v/w). The crystals were dried under reduced pressure at an outside temperature of 40°C of the reaction container for 2 hours to give (R)-3-(9H-fluoren-9-ylmethoxycarbonylamino)-3-phenyl-propanoic acid lithium salt (430 mg, 84% yield).

Mass spectrometry (MS (ESI)) was performed under the condition 1 of high-performance liquid chromatography. The peak with m/z = 388.2 $(M + H)^+$ of Fmoc-β-Phg-OH was confirmed at a retention time of 4.2 minutes. Apparent purity analysis was performed under the condition 2 of high-performance liquid chromatography. The peak of Fmoc-β-Ala-OH, one of the impurities, was confirmed at a retention time of 2.4 minutes. The peak of Fmoc-β-Phg-OH was confirmed at a retention time of 2.8 minutes. The UV absorption peak areas of Fmoc-β-Phg-OH and Fmoc-β-Ala-OH were measured at a measurement wavelength of 254 nm, and the apparent purity and the apparent content percentage of impurities were calculated according to the expressions 1b and 2b. The calculation of the apparent purity and the apparent content percentage of impurities was performed before and after Example 2-1 (before and after purification). The results are shown in Table 6.

[Table 6]

| | Fmoc-β-Ala-OH (Apparent content percentage of impurities) | Fmoc-β-Phg-OH (Apparent purity) |
|---|---|---|
| Before purification | 4.7% | 95.3% |
| After purification | 0.5% | 99.5% |

Example 2-2: Synthesis of (S)-1-(9H-fluoren-9-ylmethoxycarbonyl)pyrrolidine-2-carboxylic acid lithium salt

[Formula 7]

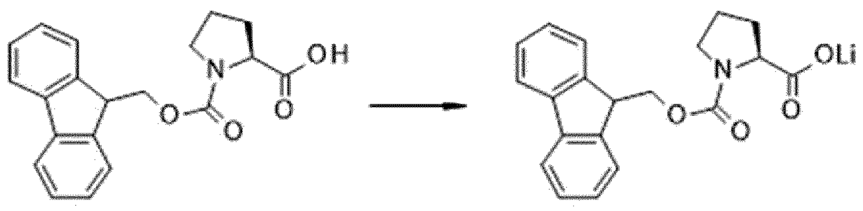

To (S)-1-(9H-fluoren-9-ylmethoxycarbonyl)pyrrolidine-2-carboxylic acid (500 mg, 1.5 mmol) and 3-(9H-fluoren-9-ylmethoxycarbonylamino)propanoic acid (23 mg, 0.074 mmol), tetrahydrofuran (6.8 ml, 14 v/w) and distilled water (0.75 ml, 1.5 v/w) were added, and the mixture was stirred at an outside temperature of 25°C of the reaction container using a stirring blade. A 4 M aqueous lithium hydroxide solution (0.37 mL, 1.5 mmol) was added over 2 minutes, and the mixture was stirred for 1 hour. Tetrahydrofuran (5.0 ml, 10 v/w) was added over 10 minutes. The mixture was stirred for 20 minutes, and precipitated crystals were confirmed. Tetrahydrofuran (7.5 ml, 15 v/w) was added over 15 minutes, and the mixture was stirred for 20 minutes. Tetrahydrofuran (5.0 ml, 10 v/w) was added over 10 minutes, and the mixture was stirred for 40 minutes. The crystals were filtered, and the obtained crystals were washed with distilled water/tetrahydrofuran (v/v) = 5/95 (7.5 mL, 15 v/w). The crystals were dried under reduced pressure at an outside temperature of 40°C of the reaction container for 2 hours to give (S)-1-(9H-fluoren-9-ylmethoxycarbonyl)pyrrolidine-2-carboxylic acid lithium salt (400 mg, 79% yield).

Mass spectrometry (MS (ESI)) was performed under the condition 1 of high-performance liquid chromatography. The peak with m/z = 338.1 $(M + H)^+$ of Fmoc-Pro-OH was confirmed at a retention time of 3.9 minutes. Apparent purity analysis was performed under the condition 2 of high-performance liquid chromatography. The peak of Fmoc-β-Ala-OH, one of the impurities, was confirmed at a retention time of 2.4 minutes. The peak of Fmoc-Pro-OH was confirmed at a retention time of 2.6 minutes. The UV absorption peak areas of Fmoc-Pro-OH and Fmoc-β-Ala-OH were measured at a measurement wavelength of 254 nm, and the apparent purity and the apparent content percentage of impurities were calculated according to the expressions 1b and 2b. The calculation of the apparent purity and the apparent content percentage of impurities was performed before and after Example 2-2 (before and after purification). The results are shown in Table 7.

[Table 7]

| | Fmoc-β-Ala-OH (Apparent content percentage of impurities) | Fmoc-Pro-OH (Apparent purity) |
|---|---|---|
| Before purification | 5.1% | 94.9% |
| After purification | lower than 0.1% | 99.9% or higher |

Example 2-3: Synthesis of (S)-2-(benzyloxycarbonyl(methyl)amino)-3-phenylpropanoic acid lithium salt

[Formula 8]

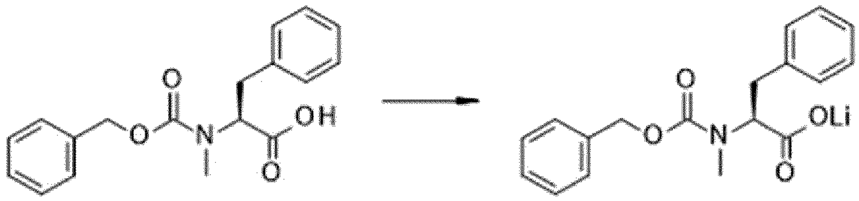

Acetonitrile (6.8 ml, 14 v/w) and distilled water (0.75 ml, 1.5 v/w) were added to (S)-2-(benzyloxycarbonyl(methyl)amino)-3-phenylpropanoic acid (500 mg, 1.6 mmol) and 3-(benzyloxycarbonylamino)propanoic acid (18 mg, 0.080 mmol), and the mixture was stirred at an outside temperature of 25°C of the reaction container using a stirring blade. A 4 M aqueous lithium hydroxide solution (0.44 ml, 1.8 mmol) were added thereto over 2 minutes. The mixture was stirred for 10 minutes, and precipitated crystals were confirmed. Acetonitrile (13 ml, 25 v/w) was added thereto over 30 minutes, and the mixture was stirred for 90 minutes. The crystals were filtered, and the obtained crystals were washed with distilled water/acetonitrile (v/v) = 5/95 (5.0 mL, 10.0 v/w). The crystals were dried under reduced pressure at an outside temperature of 40°C of the reaction container for 3 hours to give (S)-2-(benzyloxycarbonyl(methyl)amino)-3-phenylpropanoic acid lithium salt (460 mg, 90% yield).

Mass spectrometry (MS (ESI)) was performed under the condition 1 of high-performance liquid chromatography. The peak with m/z = 314.1 $(M + H)^+$ of Cbz-MePhe-OH was confirmed at a retention time of 3.9 minutes. Apparent purity analysis was performed under the condition 2 of high-performance liquid chromatography. The peak of Cbz-β-Ala-OH, one of the impurities, was confirmed at a retention time of 1.6 minutes. The peak of Cbz-MePhe-OH was confirmed at a retention time of 2.5 minutes. The UV absorption peak areas of Cbz-MePhe-OH and Cbz-β-Ala-OH were measured at a measurement wavelength of 210 nm, and the apparent purity and the apparent content percentage of impurities were calculated according to the expressions 1b and 2b. The calculation of the apparent purity and the apparent content

percentage of impurities was performed before and after Example 2-3 (before and after purification). The results are shown in Table 8.

[Table 8]

| | Cbz-β-Ala-OH (Apparent content percentage of impurities) | Cbz-MePhe-OH (Apparent purity) |
|---|---|---|
| Before purification | 2.6% | 97.4% |
| After purification | N.D. | 100% |
| N.D.: not detected | | |

Example 2-4: Synthesis of (S)-2-(tert-butoxycarbonylamino)-3-phenylpropanoic acid lithium salt

[Formula 9]

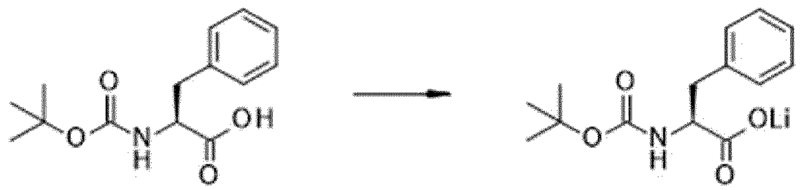

Acetonitrile (9.8 ml, 20 v/w) and distilled water (0.25 ml, 0.5 v/w) were added to (S)-2-(tert-butoxycarbonylamino)-3-phenylpropanoic acid (500 mg, 1.9 mmol) and 3-(tert-butoxycarbonylamino)propanoic acid (18 mg, 0.094 mmol), and the mixture was stirred at an outside temperature of 25°C of the reaction container using a stirring blade. Furthermore, a 1 M lithium tert-butoxide tetrahydrofuran solution (1.0 ml, 1.0 mmol) was added thereto over 5 minutes, and the mixture was stirred for 30 minutes. Acetonitrile (10 ml, 20 v/w) was added thereto over 1 hour, and the mixture was stirred for 2 hours. The crystals were filtered, and the obtained crystals were washed with distilled water/acetonitrile (v/v) = 1/99 (5.0 mL, 10.0 v/w). The crystals were dried under reduced pressure at an outside temperature of 40°C of the reaction container for 2 hours to give (S)-2-(tert-butoxycarbonylamino)-3-phenylpropanoic acid lithium salt (360 mg, 71% yield).

Mass spectrometry (MS (ESI)) was performed under the condition 1 of high-performance liquid chromatography. The peak with m/z = 166.0 $(M\text{-}Boc)^+$ of Boc-Phe-OH was confirmed at a retention time of 3.5 minutes. Apparent purity analysis was performed under the condition 1 of high-performance liquid chromatography. The peak of Boc-β-Ala-OH, one of the impurities, was confirmed at a retention time of 2.5 minutes. The peak of Boc-Phe-OH was confirmed at a retention time of 3.5 minutes. The UV absorption peak areas of Boc-Phe-OH and Boc-β-Ala-OH were measured at a measurement wavelength of 197 nm, and the apparent purity and the apparent content percentage of impurities were calculated according to the expressions 1b and 2b. The calculation of the apparent purity and the apparent content percentage of impurities was performed before and after Example 2-4 (before and after purification). The results are shown in Table 9.

[Table 9]

| | Boc-β-Ala-OH (Apparent content percentage of impurities) | Boc-Phe-OH (Apparent purity) |
|---|---|---|
| Before purification | 0.4% | 99.6% |
| After | lower than 0.1% | 99.9% or higher |
| purification | | |

**Claims**

1. A method for producing a salt of an amino acid or a salt of a peptide compound or a solvate thereof, comprising the following steps (A) and (B):
step (A); a step of contacting a lithium-containing substance with a mixture to be purified which is a mixture of the following purification target (i) and the following impurities (ii):

(i) the amino acid having a protective group at the N terminus or the peptide compound having a protective group at the N terminus, and
(ii) compounds other than the purification target, and step (B); a step of precipitating a lithium salt of the purification target.

**2.** A method for removing impurities from a mixture to be purified, comprising the following steps (A) and (B):
step (A); a step of contacting a lithium-containing substance with a mixture to be purified which is a mixture of the following purification target (i) and the following impurities (ii):

(i) an amino acid having a protective group at the N terminus or a peptide compound having a protective group at the N terminus, and
(ii) compounds other than the purification target, and step (B); a step of precipitating a lithium salt of the purification target.

**3.** The method according to claim 1 or 2, wherein the protective group at the N terminus of the purification target is a carbamate-type protective group.

**4.** The method according to any one of claims 1 to 3, wherein the purification target has a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group.

**5.** The method according to any one of claims 1 to 4, wherein the impurities are amino acids having a protective group at the N terminus or peptide compounds having a protective group at the N terminus, other than the purification target.

**6.** The method according to any one of claims 1 to 5, wherein the impurities are β-alanine having a protective group at the N terminus.

**7.** The method according to any one of claims 1 to 6, wherein the lithium salt of the purification target is formed in the step (A).

**8.** The method according to any one of claims 1 to 7, wherein the lithium salt of the purification target or a solvate thereof is precipitated as a solid in the step (B).

**9.** The method according to any one of claims 1 to 8, wherein the lithium salt of the purification target or a solvate thereof is precipitated as crystals in the step (B).

**10.** The method according to any one of claims 1 to 9, wherein the step (A) is performed in the presence of a first organic solvent.

**11.** The method according to any one of claims 1 to 10, wherein the step (B) is performed in the presence of a second organic solvent.

**12.** The method according to any one of claims 1 to 11, wherein the step (B) is performed in the presence of water.

**13.** The method according to any one of claims 1 to 12, wherein the amino acid or an N-terminal amino acid constituting the peptide compound is represented by the following general formula (1):

[Formula 1]

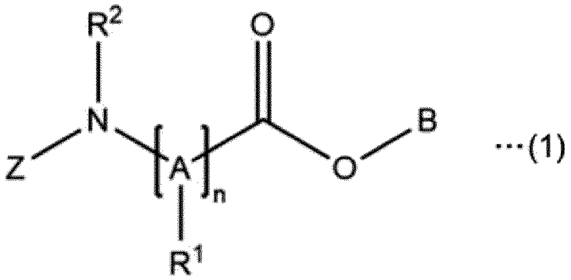

wherein

in the general formula (1),
n represents a number of 1 or more and 3 or less,
when a plurality of $R^1$ are present, $R^1$ are the same as or different from each other,
$R^1$ and $R^2$ each represent a hydrogen atom, an alkyl group, an alkenyl group, an alkynyl group, a cycloalkyl group, an aryl group, a heterocyclyl group, or a heteroaryl group, or a substituent in which two or more selected from the group consisting of these substituents are bonded, these substituents optionally have a substituent, each of these groups is optionally a saturated hydrocarbon group or an unsaturated hydrocarbon group,
A represents a carbon atom,
B represents a hydrogen atom or a binding point to an amino acid or a peptide compound,
Z represents a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group,
$R^1$ and $R^2$ are optionally bonded to form a ring together with N and A, wherein each of $R^1$ and $R^2$ is a substituent having a structure obtained by eliminating one hydrogen atom from the structure of the substituent that is not involved in ring formation, and
when n is 2, at least one of $R^2$ and two $R^1$ is not a hydrogen atom.

**14.** The method according to any one of claims 1 to 13, wherein a purity of the precipitate obtained in the step (B) is 95% by mol or higher.

**15.** A lithium salt of an amino acid or a lithium salt of a peptide compound or a solvate thereof, wherein

the lithium salt of an amino acid contains a lithium salt of an amino acid represented by the following general formula (1) with a purity of 99% by mol or higher or an apparent purity of 99% or higher, and
the lithium salt of a peptide compound contains a lithium salt of a peptide compound having a residue of an amino acid represented by the following general formula (1) with a purity of 99% by mol or higher or an apparent purity of 99%,

[Formula 2]

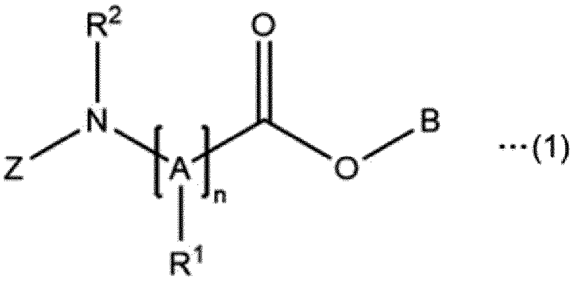

wherein

in the general formula (1),
n represents a number of 1 or more and 3 or less,

when a plurality of $R^1$ are present, $R^1$ are the same as or different from each other,
$R^1$ and $R^2$ each represent a hydrogen atom, an alkyl group, an alkenyl group, an alkynyl group, a cycloalkyl group, an aryl group, a heterocyclyl group, or a heteroaryl group, or a substituent in which two or more selected from the group consisting of these substituents are bonded, these substituents optionally have a substituent, each of these groups is optionally a saturated hydrocarbon group or an unsaturated hydrocarbon group,
A represents a carbon atom,
B represents a hydrogen atom or a binding point to an amino acid or a peptide compound,
Z represents a fluorenylmethoxycarbonyl group, a tert-butoxycarbonyl group, a benzyloxycarbonyl group, an allyloxycarbonyl group, a 2,2,2-trichloroethoxycarbonyl group, or a 2-(trimethylsilyl)ethoxycarbonyl group,
$R^1$ and $R^2$ are optionally bonded to form a ring together with N and A, wherein each of $R^1$ and $R^2$ is a substituent having a structure obtained by eliminating one hydrogen atom from the structure of the substituent that is not involved in ring formation, and
when n is 2, at least one of $R^2$ and two $R^1$ is not a hydrogen atom.

**INTERNATIONAL SEARCH REPORT**

International application No.
**PCT/JP2023/017844**

**A. CLASSIFICATION OF SUBJECT MATTER**

***C07C 269/08***(2006.01)i; ***C07C 227/28***(2006.01)i; ***C07C 229/12***(2006.01)i; ***C07C 271/22***(2006.01)i; ***C07K 1/00***(2006.01)i; ***C12N 9/06***(2006.01)i; ***C12N 15/31***(2006.01)i; ***C12N 15/53***(2006.01)i; ***C12P 13/04***(2006.01)i
FI: C07C269/08 CSP; C07K1/00 ZNA; C07C227/28; C07C229/12; C12N9/06 Z; C12N15/31; C12P13/04 ZNA; C07C271/22; C12N15/53

According to International Patent Classification (IPC) or to both national classification and IPC

**B. FIELDS SEARCHED**

Minimum documentation searched (classification system followed by classification symbols)

C07C269/08; C07C227/28; C07C229/12; C07C271/22; C07K1/00; C12N9/06; C12N15/31; C12N15/53; C12P13/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | JP 51-59889 A (MEIJI SEIKA CO.) 25 May 1976 (1976-05-25)<br>page 3, upper right column | 1-4, 7-15<br>5, 6 |
| X<br>A | JP 4-229199 A (BECTON DICKINSON & CO.) 18 August 1992 (1992-08-18)<br>paragraph [0042] | 1-4, 7-15<br>5, 6 |
| X<br>A | JP 7-501312 A (GESELLSCHAFT FUER BIOTECHNOLOGISCHE FORSCHUNG MBH) 09 February 1995 (1995-02-09)<br>page 4, lower left column to upper right column | 1-4, 7-15<br>5, 6 |
| X<br>A | JP 2004-524297 A (ELI LILLY AND CO.) 12 August 2004 (2004-08-12)<br>paragraphs [0239]-[0241] | 1-4, 7-15<br>5, 6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2023** | **25 July 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/017844**

**C. DOCUMENTS CONSIDERED TO BE RELEVANT**

| Category* | Citation of document, with indication, where appropriate, of the relevant passages | Relevant to claim No. |
|---|---|---|
| X | CN 101456859 A (SOUTH CHINA UNIVERSITY OF TECHNOLOGY) 17 June 2009 (2009-06-17) example 1 | 1-4, 7-15 |
| A | | 5, 6 |
| X | CN 105272910 A (CHENGDU XINJIE HI-TECH DEVELOPMENT CO., LTD.) 27 January 2016 (2016-01-27) example 1 | 1-4, 7-15 |
| A | | 5, 6 |
| X | CN 105273023 A (KUNMING JIDA PHARMACEUTICAL CO., LTD.) 27 January 2016 (2016-01-27) paragraph [0009] | 1-4, 7-15 |
| X | JP 2021-519766 A (UNIV. OF LEEDS) 12 August 2021 (2021-08-12) paragraphs [0402], [0403] | 1-4, 7-15 |
| A | | 5, 6 |
| A | Journal of Peptide Science. 2008, 14, 763-766, Published online 25 January 2008 in Wiley InterScience (www.interscience.wiley.com). DOI:10.1002/psc.1001 abstract, table 2 | 1-15 |

Form PCT/ISA/210 (second sheet) (January 2015)

**INTERNATIONAL SEARCH REPORT**

International application No.
**PCT/JP2023/017844**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/JP2023/017844**

| Patent document cited in search report | Publication date (day/month/year) | Patent family member(s) | Publication date (day/month/year) |
|---|---|---|---|
| JP 51-59889 A | 25 May 1976 | (Family: none) | |
| JP 4-229199 A | 18 August 1992 | US 5055594 A<br>example 1<br>EP 467318 A1 | |
| JP 7-501312 A | 09 February 1995 | WO 1992/022566 A1<br>pp. 8, 9<br>EP 589927 A1 | |
| JP 2004-524297 A | 12 August 2004 | US 2004/0116699 A1<br>manufacturing example C3<br>WO 2002/059095 A1<br>EP 1358163 A1 | |
| CN 101456859 A | 17 June 2009 | (Family: none) | |
| CN 105272910 A | 27 January 2016 | (Family: none) | |
| CN 105273023 A | 27 January 2016 | (Family: none) | |
| JP 2021-519766 A | 12 August 2021 | WO 2019/186164 A1<br>pp. 118, 119<br>EP 3773555 A1<br>KR 10-2020-0135846 A<br>CN 112165938 A<br>US 2022/0305011 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001060795 A
- WO 1999033785 A


### Non-patent literature cited in the description


- **M. OBKIRCHER et al.** Formationof Fmoc-β-alanine during Fmoc-protections with Fmoc-Osu. *Journal ofPeptide Science*, June 2008, vol. 14 (6), 763-766
- **KARLIN** ; **ALTSCHUL**. *Proc. Natl. Acad. Sci. USA*, 1993, vol. 90, 5873-7
- **ALTSCHUL et al.** *J. Mol. Biol.*, 1990, vol. 215, 403-10
- **HOPP, T.P. et al.** *BioTechnology*, 1988, vol. 6, 1204-1210